(19) Europäisches Patentamt
European Patent Office
Office européen des brevets

(11) **EP 2 572 633 A2**

(12) **EUROPÄISCHE PATENTANMELDUNG**

(43) Veröffentlichungstag:
**27.03.2013 Patentblatt 2013/13**

(21) Anmeldenummer: **12006755.8**

(22) Anmeldetag: **20.08.2010**

(51) Int Cl.:
**A61B 5/0205** (2006.01)  **A61B 5/021** (2006.01)
**A61B 5/024** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **28.08.2009   DE 102009039257**

(62) Dokumentnummer(n) der früheren Anmeldung(en) nach Art. 76 EPÜ:
**10749607.7 / 2 348 972**

(71) Anmelder: **UP-MED GmbH**
**81673 München (DE)**

(72) Erfinder:
• **Pfeiffer, Ulrich**
**81667 München (DE)**

• **Thomamüller, Tobias**
**83052 Bruckmühl (DE)**
• **Knoll, Reinhold**
**94127 Neuburg (DE)**

(74) Vertreter: **Graf von Stosch, Andreas et al**
**Graf von Stosch**
**Patentanwaltsgesellschaft mbH**
**Prinzregentenstrasse 22**
**80538 München (DE)**

Bemerkungen:
•Diese Anmeldung ist am 27-09-2012 als Teilanmeldung zu der unter INID-Code 62 erwähnten Anmeldung eingereicht worden.
•Die Patentansprüche wurden nach dem Anmeldetag eingereicht/nach dem Tag des Eingangs der Teilanmeldung eingereicht (R. 68(4) EPÜ).

(54) **Blutdruckmessvorrichtung und Verfahren zur Blutdruckmessung eines Lebewesens**

(57)      Die Erfindung betrifft eine Vorrichtung und ein Verfahren zur nicht-invasiven Messung von pulsatilen Signalen eines Lebewesens, bevorzugt eines Menschen.

Die Vorrichtung umfasst ein flexibles Element, das eingerichtet ist, ein Körperteil zumindest teilweise zu umgeben, wobei an dem flexiblen Element mindestens ein Drucksensorelement angebracht ist und das flexible Element eingerichtet ist, dem Oberflächenprofil des Körperteils angepasst zu werden und das flexible Element eine Versteifungseinrichtung aufweist, durch die das flexible Element versteifbar ist.

Bei dem erfindungsgemäßen Verfahren wird zunächst das flexible Element mit einem Drucksensorelement an ein für die Messung relevantes Körperteil angelegt, so dass das flexible Element eine körperteilangepasste Form einnimmt, daraufhin wird das flexible Element in der körperteilangepassten Form versteift, anschließend wird das Drucksignal über einen bestimmten Zeitraum während sich das flexible Element in dem versteiften Zustand befindet gemessen und abschließend erfolgt die Überführung des Zustands des flexiblen Elements zurück in den flexiblen Zustand.

fig 4

EP 2 572 633 A2

**Beschreibung**

Die Erfindung betrifft eine Vorrichtung und ein Verfahren zum Blutdruckmessen eines Lebewesens. Insbesondere betrifft die Erfindung eine Vorrichtung und ein Verfahren zur Bestimmung von Parametern der Herz-Lungen-Interaktion eines Lebewesens.

In der medizinischen Praxis ist es in vielen Situationen notwendig, Informationen über den Zustand des Kreislaufsystems zu erhalten. Insbesondere bedarf es in der klinischen Medizin oftmals bestimmter Messgrößen, die Entscheidungen über eine gezielte Beeinflussung des Herzkreislaufsystems erlauben, beispielsweise inwieweit eine Auffüllung des Kreislaufs mittels Infusionslösung sinnvoll ist, oder ob alternativ bzw. in welchem Ausmaß der Kreislauf durch kreislauf-aktive Medikamente unterstützt werden sollte. Hierfür haben sich besonders Parameter der Herz-Lungen-Interaktion (HLI) als nützlich erwiesen. Diese beruhen jedoch meist auf einer invasiven Messung des arteriellen Blutdrucks und erfordern eine aufwendige Kanülierung eines arteriellen Gefäßes.

Nicht-invasive Methoden zur Messung von HLI-Parametern sind bisher aufgrund der schlechteren Signalqualität nicht sehr leistungsfähig. Die pulsatilen Signale, d.h. die durch den Puls herbeigeführten Druckänderungen in den arteriellen Gefä-βen, werden mittelbar über das Gewebe mit einer externen Vorrichtung gemessen. Hierbei wirken sich Dämpfungseffekte, verursacht durch Dämpfung im Gewebe und in der Messvorrichtung, erheblich negativ auf das Signal-Rausch-Verhältnis aus. In der Praxis scheitern nicht-invasive Methoden daher.

In der US 2005/187481 A1 werden Methoden zur Bestimmung von HLI-Parametern angegeben. Auch auf eine nicht-invasive Messung mittels Finger-, Armmanschetten oder Ohrklammern wird hingewiesen. Wie mit diesen Messinstrumenten eine geeignete Signalqualität erreicht werden kann ist jedoch nicht offenbart.

Das US-Patent US 5 255 686 offenbart eine Vorrichtung zur nicht-invasiven, kontinuierlichen Blutdruckmessung. Als Messkomponente dient eine konventionelle Blutdruckmanschette, mit welcher der systolische, diastolische und mittlere Blutdruck bestimmt wird.

Aufgabe der vorliegenden Erfindung war es, eine Vorrichtung und ein Verfahren zum Messen von Blutdruck bereitzustellen, welche die Signalqualität bei nicht-invasiver Messung von pulsatilen Signalen gegenüber dem Stand der Technik verbessern.

Die Aufgabe wird durch die unabhängigen Ansprüche gelöst. Vorteilhafte Weiterbildungen sind in den Unteransprüchen definiert.

Insbesondere wird die Aufgabe gelöst durch eine Blutdruckmessvorrichtung (20) umfassend ein flexibles Element (30), das eingerichtet ist, ein Körperteil (10) zumindest teilweise zu umgeben, wobei an dem flexiblen Element (30) mindestens ein Drucksensorelement (40) angebracht ist und das flexible Element (30) eine Versteifungseinrichtung (31) aufweist, durch die das flexible Element (30) versteifbar ist.

Die Blutdruckmessvorrichtung ist eine Vorrichtung, mit welcher pulsatile Signale, d.h. Druckoszillationen in blutführenden Gefäßen eines Lebewesens, bevorzugt eines Menschen, gemessen werden können. Sie ist zur nicht-invasiven Anwendung vorgesehen.

Als flexibles Element kann bevorzugt eine Manschette eingesetzt werden, bevorzugt eine wickelbare Manschette oder eine geschlossene röhrenartige Manschette. Bevorzugt weist das flexible Element ein Kompressionselement auf, mit der das flexible Element an ein Oberflächenprofil eines räumlichen Objekts, bevorzugt an ein Oberflächenprofil eines Körperteils eines Lebewesens, besonders bevorzugt an ein Oberflächenprofil eines Körperteils eines Menschen anpassbar ist. Das Profil mindestens einer Grenzfläche des angepassten flexiblen Elements entspricht dann im Wesentlichen dem inversen Abbild des Oberflächenprofils, an welches das flexible Element angepasst wurde. Das flexible Element ist bevorzugt verformbar. Bevorzugt ist das flexible Element elastisch oder plastisch verformbar. Das flexible Element lässt sich besonders bevorzugt verbiegen und/ oder eindrücken und/ oder verwinden. Durch die Verformung des flexiblen Elements ist es möglich, mindestens eine Oberfläche des flexiblen Elements durch Überlagerung von lokal konkaven und/ oder konvexen Profilstrukturen mit einem beliebigen Oberflächenprofil zu versehen. Die Verformung des flexiblen Elements ist bevorzugt passiv, d.h. sie erfolgt durch eine oder mehrere auf das flexible Element einwirkende Kraft bzw. Kräfte. Sie wird bevorzugt durch Aufdrücken des flexiblen Elements an das Oberflächenprofil erzeugt. Das Aufdrücken erfolgt bevorzugt manuell bzw. durch eine Druckvorrichtung, besonders bevorzugt durch eine pneumatische Druckvorrichtung. Bevorzugt umschließt das flexible Element das Körperteil teilweise, besonders bevorzugt umschließt das flexible Element das Körperteil ganz. Beispielsweise lässt sich das flexible Element flächig um den Ober- oder Unterschenkel, um das Handgelenk, einen Finger oder den Oberarm eines Menschen legen, so dass es sich der jeweiligen Oberfläche anpasst und das jeweilige Körperteil teilweise oder ganz bedeckt oder umschließt. Die Abmessungen des flexiblen Elements sind bevorzugt an das entsprechende Körperteil angepasst, für das die Blutdruckmessvorrichtung vorgesehen ist. Bevorzugt besitzt das flexible Element die Form eines geschlossenen Zylindermantels. Besonders bevorzugt besitzt das flexible Element die Form einer Matte mit bevorzugt rechteckiger Mattenfläche, die zu einem Zylindermantel umgeformt werden kann.

An dem flexiblen Element ist ein Drucksensorelement angebracht. Es kann Drücke und Druckschwankungen erfassen und in elektrische Signale umwandeln. Diese Signale werden mit Hilfe eines Sensorkabels oder bevorzugt per Funk zur weiteren Verarbeitung übertragen.

Die Anbringung des Drucksensorelements ist bevorzugt derart, dass das Drucksensorelement auf einer der Oberflächen des flexiblen Elements aufliegt. Bevorzugt ist das Drucksensorelement an einer der Oberflächen des flexiblen Elements fixiert. Besonders bevorzugt ist das Drucksensorelement in eine der Oberflächen des flexiblen Elements eingearbeitet. Die bevorzugte Oberfläche für die Anbringung ist die Innenseite des flexiblen Elements. Die Innenseite ist dabei die dem Körperteil zugewandte Seite. Bevorzugt lässt sich das angebrachte Drucksensorelement abnehmen und wieder anbringen. Hierfür weist das Drucksensorelement bevorzugt ein Verbindungselement wie einen Klettverschluss, einen Saugnapf, Klebeflächen oder Druckknöpfe auf. Dieses Verbindungselement ist bevorzugt zwischen dem flexiblen Element und dem Drucksensorelement angeordnet. Besonders bevorzugt befindet sich das Verbindungselement an der der Körperoberfläche abgewandten Oberfläche des Drucksensorelements. Bei bevorzugter Anbringung des Drucksensorelements auf der Innenseite des flexiblen Elements steht es in direktem Kontakt zu dem jeweiligen Körperteil. Durch Aufdrücken des flexiblen Elements an das Körperteil wird das Drucksensorelement an das Körperteil gepresst und dadurch das Signal-Rausch-Verhältnis beim Erfassen von Messwerten verbessert.

Die Blutdruckmessvorrichtung weist bevorzugt mehrere Drucksensorelemente auf. Bevorzugt liegen die Drucksensorelemente auf einer oder mehreren Achsen mit jeweils bekannten Abständen zueinander. So kann beispielsweise die Pulswellengeschwindigkeit in einer Arterie gemessen werden, wenn mindestens zwei Drucksensorelemente auf der Innenseite des flexiblen Elements so angebracht sind, dass sie auf einer Achse liegen, die im Wesentlichen parallel zu einer Arterie des Körperteils verläuft und jeweils der Abstand von einem Drucksensorelement zum nächsten Drucksensorelement bekannt ist.

Das flexible Element weist eine Versteifungseinrichtung auf. Sie ist eingerichtet, das flexible Element zu versteifen, so dass es nicht mehr verformbar ist. Bevorzugt ist die Versteifungseinrichtung eingerichtet, das flexible Element so zu versteifen, dass es nicht mehr elastisch oder plastisch verformbar ist. Besonders bevorzugt ist die Versteifungseinrichtung eingerichtet, das flexible Element so zu versteifen, dass es sich nicht mehr verbiegen und/ oder eindrücken und/oder verwinden lässt. Durch Versteifung mit Hilfe der Versteifungseinrichtung wird das flexible Element bevorzugt inkompressibel. Bevorzugt wird die Form des flexiblen Elements durch die Versteifungseinrichtung beim Versteifen nicht wesentlich verändert, so dass das Oberflächenprofil des flexiblen Elements im Wesentlichen erhalten bleibt. Bevorzugt bleibt das an das Körperteil eines Lebewesens, bevorzugt eines Menschen, angepasste Oberflächenprofil des flexiblen Elements beim Versteifen erhalten. Bevorzugt ist die Versteifungseinrichtung im Wesentlichen in der gesamten räumlichen Ausdehnung des flexiblen Elements vorhanden. Bevorzugt ist die Versteifungseinrichtung im flexiblen Element im Wesentlichen gleichmäßig integriert, so dass die Versteifungseinrichtung das flexible Element im Wesentlichen gleichmäßig versteift.

Beispielsweise können allein mehrere Lagen von Blättern aus Papier oder aus einem ähnlichen Material eine Versteifungseinrichtung darstellen. Bereits ab ca. 20 aufeinander gelegten Blättern mit einer bevorzugten Stärke von 80 g/m$^2$ kann eine Versteifung der Blätter durch eine Wölbung des Stapels herbeigeführt werden. Der Stapel lässt sich nicht mehr eindrücken. Dieser Effekt ist beispielsweise bei einem aus mehreren Lagen Papier geformten Zylinder zu beobachten. Bei Verwendung eines solches Stapels als Versteifungseinrichtung sind besonders bevorzugt 50 ± 10 Blätter Papier (bevorzugt 80 g/m$^2$) in dem Stapel enthalten. Beispielsweise wird ein solcher Stapel Papier um eine Extremität gewickelt und wird dadurch steif.

Die Versteifungseinrichtung ist bevorzugt über eine Steuerleitung aktivierbar, wodurch das flexible Element versteift wird. Bevorzugt ist die Versteifungseinrichtung über eine Steuerleitung auch wieder deaktivierbar, wodurch das flexible Element wieder verformbar wird.

Durch die erfindungsgemäße Blutdruckmessvorrichtung wird eine Dämpfung der pulsatilen Signale durch Komponenten der Vorrichtung und/ oder durch Luft außerhalb der Vorrichtung im Wesentlichen verhindert. Dadurch wird eine hohe Signalqualität erreicht. Dies ist möglich durch die hydraulisch optimierte Ankopplung des Drucksensorelements an das für die Messung relevante Körperteil.

Durch die hydraulisch optimierte Ankopplung des an das Körperteil angepasste Oberflächenprofil des flexiblen Elements wird der Kontakt des Drucksensorelements zu dem Körperteil verbessert.

In einem weiteren Ausführungsbeispiel der vorliegenden Erfindung ist eine Blutdruckmessvorrichtung vorgesehen, bei der das Drucksensorelement (40) ein in ein Gelkissen (44) eingebetteter Drucksensor (41) ist.

Ein Gelkissen besteht bevorzugt aus einer abgedichteten Hülle, in deren Hohlraum eine Füllung vorgesehen ist. Die Füllung ist bevorzugt ein Fluid mit einer Viskosität zwischen 0,1 mPa s und 10$^7$ mPa s, besonders bevorzugt zwischen 0,6mPa s und 10$^6$mPa s. Besonders bevorzugt ist das Fluid ein Silikon-Gel.

Das Gelkissen lässt sich bevorzugt an das Oberflächenprofil eines Körperteils anpassen. Diese Anpassung erfolgt bevorzugt durch bloßes Aufdrücken des Gelkissens an die Oberfläche des Körperteils. Das Profil der Grenzfläche des angepassten flexiblen Elements entspricht dann im Wesentlichen dem inversen Abbild des Oberflächenprofils, an welches das flexible Element angepasst wurde. Die Anpassung ist möglich, da das Gelkissen verformbar ist. Bevorzugt ist das Gelkissen elastisch verformbar. Das Gelkissen lässt sich besonders bevorzugt verbiegen und/ oder eindrücken und/ oder verwinden.

Der Drucksensor ist ein Sensor, der Druckoszillationen im Gelkissen in elektrische Signale umwandelt. Der Drucksensor liegt bevorzugt direkt in dem Fluid. Bevorzugt befindet sich der Drucksensor im Mittelpunkt des Gelkissens.

Durch die Lagerung eines Drucksensors in ein mit Gel oder einer anderen Flüssigkeit gefülltes Kissen ist eine optimierte Ankopplung von Drucksensorelement und Körperteil möglich. Bevorzugt wird das Gelkissen durch Kontakt, bevorzugt durch Hautkontakt, mit dem Körperteil großflächig gekoppelt. Dadurch werden Druckschwankungen, die von einem Blutgefäß des Körperteils ausgehen, zu dem das Gelkissen Kontakt hat, im Wesentlichen ungedämpft auf das Gelkissen übertragen. Die Druckschwankungen können sich dann aufgrund des Fluids in dem Kissen ungehindert ausbreiten und durch den Sensor gemessen werden. Da das Gelkissen ein Drucksensorelement darstellt, das an dem flexiblen Element angebracht ist, werden die Druckschwankungen nicht mehr an weitere Komponenten weitergegeben, sofern das flexible Element versteift ist. Somit geht die Energie der Druckwellen nicht in benachbarte Komponenten der Blutdruckmessvorrichtung oder die Luft über und steht im Wesentlichen vollständig zur Messung zur Verfügung. Bevorzugt wird hierbei mehr Signalenergie vom Körperteil auf das Drucksensorelement übertragen. Damit wird das Signalrauschverhältnis verbessert. Je größer die Kontaktfläche mit dem Körperteil ist, umso größer ist die Übertragungsfläche und damit auch die Signalenergie, die für die Messung zur Verfügung steht.

In einem weiteren Ausführungsbeispiel der vorliegenden Erfindung ist eine Blutdruckmessvorrichtung vorgesehen, bei der zusätzlich weitere Sensoren vorgesehen sind, insbesondere eine Sensoreinrichtung zur Messung der Impedanz und/ oder Elektroden.

Die weiteren Sensoren sind bevorzugt an einer oder mehreren beliebigen Stellen der Blutdruckmessvorrichtung vorgesehen, besonders bevorzugt an der Innenseite des flexiblen Elements. Ganz besonders bevorzugt sind die Sensoren direkt auf einem Gelkissen angebracht in dem ein Drucksensor eingebettet ist.

Die Sensoren sind bevorzugt Elektroden zur Impedanz- und/oder Potentialmessung und/ oder photoelektrische Detektions- oder Anregungselemente und/ oder kapazitiv messende Sensoren und/ oder Beschleunigungssensoren und/ oder Streifenelektroden. Sie sind bevorzugt eingerichtet, weitere Biosignale zu messen.

So kann beispielsweise allgemein über den Widerstand, bevorzugt über den Wechselstromwiderstand des Körperteilabschnittes der pulsatile Blutfluss gemessen werden, wenn mindestens zwei Elektroden verwendet werden, wobei eine (zumindest temporär) als Anregungs- und die andere (ebenfalls zumindest temporär) als Detektionselektrode verwendet wird. Beispielsweise sind zur Impedanzmessung vier Elektroden oder Streifenelektroden (z.B. ein Metallstreifen oder ein Streifen eines flexiblen Leitermaterials) zirkulär auf der Innenseite des flexiblen Elements angebracht - also so, dass die Elektroden das Körperteil umspannen, wenn die Blutdruckmessvorrichtung an ein Körperteil angelegt ist. Vorzugsweise sind dabei die äußeren zwei Elektroden zur Injektion eines Stroms, bevorzugt eines Wechselstroms bis bevorzugt 100 mA vorgesehen. Die inneren Elektroden sind dann zur hochohmigen Messung des Impedanzsignals vorgesehen. Da sich das Volumen des erfassten Körperteilabschnittes durch pulsatiles Einströmen des elektrischen Leiters Blut verändert, kann ein mit dem pulsatilen arteriellen Blutfluss im erfassten Körperabschnitt (Messsegment) synchron verlaufendes Signal erfasst werden, so denn Blutfluss vorhanden. Pulsatile Signale können gemessen werden, wenn von außen auf das Körperteil ein Druck unterhalb des diastolischen Blutdrucks anliegt (z.B. durch eine Blutdruckmanschette). Stoßartige pulsatile, aber wieder auf Null abfallende Signale werden bei einem äußeren Druck zwischen diastolischem und systolischem Blutdruck gemessen.

Bei Verwendung von Elektroden ist bevorzugt leitendes Gel jeweils zwischen einer Elektrode und dem Körperteil vorgesehen, das den Übergangwiderstand zum Körperteil reduziert. Zur Übertragung der Sensorsignale ist bevorzugt ein Sensorkabel oder besonders bevorzugt eine Funkeinrichtung vorgesehen. Wenn ein Sensor die Funktion der Druckmessung erfüllt, kann er bevorzugt ein Drucksensorelement auch ersetzen.

In einem weiteren Ausführungsbeispiel der vorliegenden Erfindung ist eine Blutdruckmessvorrichtung vorgesehen, bei der eine äußere Fixierung (50) vorgesehen ist, die zumindest teilweise um das flexible Element (30) angeordnet ist bzw. anordenbar ist.

Die äußere Fixierung ist bevorzugt eine Druckeinrichtung bzw. eine Kompressionseinrichtung. Besonders bevorzugt ist als äußere Fixierung eine Zugeinrichtung vorgesehen. Beispielsweise ist die äußere Fixierung ein Gurtsystem mit einer oder mehreren Gurtschnallen oder Klettverschlüssen, bevorzugt ein System aus einem oder mehreren Raststreifen oder besonders bevorzugt Verschlussschnallen (wie beispielsweise bei Skischuhen verwendet). Die äußere Fixierung ist ganz besonders bevorzugt ein elastisches Element, das bevorzugt mit Material, bevorzugt einem Fluid oder Gas bevorzugt über mindestens einen Anschlussschlauch befüllbar, bevorzugt schnell befüllbar, und entleerbar ist und so bevorzugt sein Volumen änderbar ist. Besonders bevorzugt ist ein solches elastisches Element eine konventionelle Blutdruckmanschette. Weiterhin ganz besonders bevorzugt ist die äußere Fixierung eine Kombination von verschiedenen der genannten Ausführungsformen. Die äußere Fixierung ist bevorzugt wickelbar, besonders bevorzugt manschetten- oder strumpfartig. Bevorzugt besitzt die äußere Fixierung die Form eines geschlossenen Zylindermantels. Besonders bevorzugt besitzt das elastische Element die Form einer Matte mit bevorzugt rechteckiger Mattenfläche, welche die Form eines Zylindermantels einnehmen kann.

Bevorzugt ist die äußere Fixierung an der Außenseite des flexiblen Elements vorgesehen. Bevorzugt ist die äußere Fixierung an einem oder mehreren Punkten zumindest temporär fest mit dem flexiblen Element verbunden. Die Verbindung ist bevorzugt eine oder eine Kombination der Verbindungsmöglichkeiten: Klebstoff, Vernähung, Vulkanisierung, ein oder mehrere Druckknöpfe, ein oder mehrere Klettverschlüsse. Besonders bevorzugt ist die äußere Fixierung durch eine durch die äußere Fixierung erzeugte Kraft mit dem flexiblen Element verbindbar. Die äußere Fixierung ist an dem flexiblen Element an einer oder bevorzugt mehreren der Begrenzungen des flexiblen Elements bevorzugt überlappend vorgesehen, besonders bevorzugt an einer oder mehreren der Begrenzungen des flexiblen Elements bündig mit dem flexiblen Element abschließend vorgesehen. Bevorzugt ist die äußere Fixierung an dem flexiblen Element nur teilweise bedeckend vorgesehen.

Bevorzugt ist die äußere Fixierung und das flexible Element in einer Einheit verwirklicht. Diese kann bevorzugt wieder verwendbar ausgestaltet sein. Die äußere Fixierung weist bevorzugt das Merkmal auf, eine Kraft und damit einen Druck auf das flexible Element auszuüben, die bevorzugt das flexible Element gegen das Körperteil drückt. Bevorzugt wird durch diese Kraft das flexible Element an dem Körperteil fixiert. Bevorzugt greift die Kraft an der Außenseite des flexiblen Elements an, d.h. an der dem Körperteil abgewandten Oberfläche des flexiblen Elements. Bevorzugt ist die Orientierung der von der äußeren Fixierung ausgeübten Kraft senkrecht zur Oberfläche, bevorzugt senkrecht zur Außenseite des flexiblen Elements und wirkt in Richtung des Körperteils. Bevorzugt erzeugt die äußere Fixierung eine Flächenkraft, die bevorzugt senkrecht zu jedem Punkt der Oberfläche des flexiblen Elements, bevorzugt senkrecht zu jedem Punkt der Außenseite des flexiblen Elements wirkt. Bevorzugt drückt die äußere Fixierung das flexible Element auf das Körperteil, bevorzugt so, dass das flexible Element an dem Körperteil durch den Druck fixiert ist, besonders bevorzugt so dass sich die dem Körperteil zugewandte Innenseite des unversteiften flexiblen Elements dem Oberflächenprofil des Körperteils anpasst. Ganz besonders bevorzugt drückt die äußere Fixierung das unversteifte flexible Element so auf das Körperteil, dass im Wesentlichen keine Lufteinschlüsse zwischen dem flexiblen Element und dem Körperteil verbleiben, besonders bevorzugt im Wesentlichen keine Lufteinschlüsse zwischen einem oder mehreren der an der Innenseite des flexiblen Elements angebrachten Drucksensorelemente. Bevorzugt werden das Drucksensorelement oder die angebrachten Drucksensorelemente sowie bevorzugt weitere Sensoren, durch die Kraft der äußeren Fixierung zusammen mit dem flexiblen Element an das Körperteil gedrückt.

Bevorzugt ist der Grad der Fixierung, also die Kraft, mit welcher die Fixierung das flexible Element auf das Körperteil drückt, steuerbar oder automatisch regelbar vorgesehen. Zur Steuerung ist bevorzugt eine Steuerleitung vorgesehen. Bei bevorzugter Ausführung der äußeren Fixierung als elastisches Element oder besonders bevorzugt als Blutdruckmanschette ist diese Steuerleitung bevorzugt ein Schlauch, der Luft oder ein Fluid in das bevorzugte elastische Element oder die besonders bevorzugte Blutdruckmanschette über bevorzugt eine Pumpe leitet. Als allgemeiner Ersatz für eine Pumpe kann bevorzugt auch ein Drucktank dienen, der eine ausreichende Kapazität besitzt und bevorzugt in Phasen der Nicht-Messung wieder mit Druck entweder von extern oder durch eine interne Pumpe beaufschlagt wird.

Für die Bestimmung des für die Regelung notwendigen Messwerts des Fixierungsgrads ist bevorzugt ein Drucksensorelement vorgesehen, das bevorzugt den Anpressdruck zwischen dem flexiblen Element und dem Körperteil misst. Besonders bevorzugt ist dieses Drucksensorelement eines oder eine Gruppe mehrerer der bereits für die Messung der pulsatilen Signale vorgesehenen Drucksensorelemente.

In einem weiteren Ausführungsbeispiel der vorliegenden Erfindung ist eine Blutdruckmessvorrichtung (20) vorgesehen, bei der das Drucksensorelement (40) ein Sensor oder eine Kombination von Sensoren aus der Menge folgender Sensoren ist: Elektroden (42) zur Impedanz- und/ oder Potentialmessung, photoelektrische Sensoren, kapazitive Sensoren, Beschleunigungssensoren

Die Kombination der Sensoren weist bevorzugt verschiedene, besonders bevorzugt nur gleichartige Sensoren auf. Ein Drucksensorelement weist bevorzugt eine beliebige Anzahl dieser Sensoren auf.

In einem weiteren Ausführungsbeispiel der vorliegenden Erfindung ist eine Blutdruckmessvorrichtung (20) vorgesehen, bei der die Versteifungseinrichtung (31) mindestens eine luftdichte Hülle (32) mit mindestens je einem Anschlussschlauch (36) aufweist.

Die luftdichte Hülle besteht bevorzugt aus einem Material, das als Eigenschaft Flexibilität, bevorzugt eine hohe Luftundurchlässigkeit besitzt. Bevorzugt umschließt sie einen Hohlraum. Die luftdichte Hülle ist bevorzugt röhren- bzw. schlauchförmig. Besonders bevorzugt ist die luftdichte Hülle mattenartig mit bevorzugt rechteckiger Mattenfläche. Ganz besonders bevorzugt weist die luftdichte Hülle näherungsweise die Form einer Torusfläche auf. Dabei handelt es sich bevorzugt um einen flachen Torus. Die luftdichte Hülle ist bevorzugt zusammen mit dem flexiblen Element verformbar. Bevorzugt befinden eine oder mehrere der luftdichten Hüllen innerhalb des flexiblen Elements. Wenn mehrere luftdichte Hüllen vorhanden sind, sind die luftdichten Hüllen bevorzugt in gleichmäßiger Verteilung angeordnet.

Weiterhin bevorzugt besteht die luftdichte Hülle aus zwei Lagen sehr dehnungssteifen Materials (z.B. gummiertes Gewebe oder ein Material ähnlich eines Auto- oder Fahrradreifens), die miteinander luftdicht verbunden (z.B. verschweißt oder vulkanisiert) sind. Die Verbindung begrenzt mindestens eine luftdichte, bevorzugt längliche Kammer. In dieser Ausführungsform ist die Dicke des flexiblen Elements vorzugsweise nicht größer als die beiden verbundenen Lagen Material. Die Lagen sind bevorzugt vororientiert miteinander verbunden, sodass bei Füllung der mindestens einen Kammer mit Druckluft eine dem Körperteil (z.B. Arm) angepasste Form des flexiblen Elements entsteht.

Der Anschlussschlauch ist bevorzugt vorgesehen, Luft oder ein spezielles Gas oder eine Flüssigkeit in die luftdichte Hülle einzupumpen oder zu evakuieren.

Bevorzugt verkleinert sich der Hohlraum beim Evakuieren der Hülle oder verschwindet komplett. Bei Verwendung eines mit Gas befüllbaren elastischen Elements als äußere Fixierung und insbesondere bei Verwendung einer konventionellen Blutdruckmanschette als äußere Fixierung werden bevorzugt die Steuerleitung der äußeren Fixierung, welche in diesem Fall ein Schlauch ist, und der Anschlussschlauch der luftdichten Hülle derart miteinander verbunden oder besonders bevorzugt verbindbar ausgelegt, dass Luft oder das spezielle Gas zwischen der äußeren Fixierung und der luftdichten Hülle ausgetauscht werden kann. Bevorzugt führt dann eine Entlüftung des flexiblen Elements zur Belüftung der äußeren Fixierung und/ oder vice versa. Bevorzugt ist eine mindestens doppelte Ausführung der Anschlussschläuche vorgesehen, so dass für Be- und Entlüftung der luftdichten Hülle jeweils mindestens ein Schlauch zur Verfügung steht. Damit lässt sich die für den Luftaustausch benötigte Zeit verringern.

Ein Anschlussschlauch weist bevorzugt mindestens ein Ventil auf, durch welches Materialströme bevorzugt in beliebiger Richtung begrenzt oder besonders bevorzugt ganz verhindert werden können.

In einem weiteren Ausführungsbeispiel der vorliegenden Erfindung ist eine Blutdruckmessvorrichtung (20) vorgesehen, bei der die luftdichte Hülle (32) im Wesentlichen inkompressible Elemente aufweist, deren Volumen sich in einem Vakuum um weniger als 50% gegenüber dem Volumen bei Atmosphärendruck ändert.

Bevorzugt ändert sich das Volumen dieser Elemente um weniger als 25%, besonders bevorzugt um weniger als 10%, ganz besonders bevorzugt um weniger als 1%. Idealerweise geht die Änderung des Volumens im Vakuum gegenüber dem Volumen bei Atmosphärendruck gegen Null.

Bevorzugt füllen eine Vielzahl dieser im Wesentlichen inkompressiblen Elemente die luftdichte Hülle aus. Die Form der Elemente ist bevorzugt kugelförmig, eckig, oder eine Mischung aus beidem. Es ist eine Vielzahl von hierfür geeigneten Elementen denkbar. Bevorzugt sind diese Elemente sehr klein (z.B. im Bereich von der Größe von Elementen eines Pulvers bis zu der Größe von Kastanien) und sie lassen sich bevorzugt im Wesentlichen beliebig zueinander anordnen. Bei Kompression des Verbunds dieser Elemente verhaken, verkeilen oder verpressen sich diese Elemente vorzugsweise und die Anordnung der Elemente erstarrt sozusagen.

Die im Wesentlichen inkompressiblen Elemente sind zum Beispiel Kunststoffgranulat, Reis, Körper aus Polistyrol oder ähnlichem Kunststoff, Papierfetzen, Papierkügelchen, Papierblätter, Styroporkugeln, Sägemehl, Salz, beliebige Pulver oder ähnliche Elemente. Die in der luftdichten Hülle befindlichen, im Wesentlichen inkompressiblen Elemente sind bevorzugt eine Mischung verschiedenartiger inkompressibler Elemente. Besonders bevorzugt sind als inkompressible Elemente Blätter aus Papier oder einem anderen Material, die zu einem Stapel geschichtet sind, so wie der zuvor bereits beschriebene Stapel Papier als Realisierung einer Versteifungseinrichtung. Der Unterschied ist lediglich, dass sich der genannte Stapel Papier in der luftdichten Hülle befindet.

Weiterhin beispielhaft ist eine magnetorheologische Flüssigkeit geeigneter Zusammensetzung als Füllung der luftdichten Hülle möglich. Die inkompressiblen Elemente sind dabei kleine, magnetisch polarisierbare Partikel (z.B. Eisenpulver), die in einer Suspension schwimmen. Magnetorheologische Flüssigkeiten sind durch den Stand der Technik bekannt. Bevorzugt reicht in diesem Fall aus, wenn die beschriebene luftdichte Hülle nicht unbedingt luftdicht, sondern nur flüssigkeitsdicht bezüglich der magnetorheologischen Flüssigkeit ist. In diesem Fall ist zudem der Anschlussschlauch bevorzugt nicht vorhanden. Durch Anlegen eines Magnetfeldes (z.B. Anschalten eines die Fläche der Versteifungseinrichtung erfassenden Elektromagneten-Arrays oder durch Umwicklung des flexiblen Elements bzw. der Blutdruckmessvorrichtung mit mindestens einem Elektro- und/ oder Permanentmagnetenverbund, der beispielsweise mattenartig realisiert ist, oder auch nur unter Anwendung eines einzelnen Magneten und/ oder Elektromagneten) eine bevorzugt schlagartige Versteifung der Versteifungseinrichtung erreicht werden. Die Flüssigkeit behält im Wesentlichen die zuvor eingenommene Form. Bei Abschalten bzw. Entfernung des Magnetfeldes erfolgt bevorzugt eine ebenso schnelle Entsteifung.

In einem weiteren Ausführungsbeispiel der vorliegenden Erfindung ist eine Blutdruckmessvorrichtung (20) vorgesehen, bei der die luftdichte Hülle (32) vernetzte Fasern aufweist.

Die Fasern sind bevorzugt dünne, bevorzugt gekräuselte, Fäden eines bevorzugt beliebigen Materials.

Die Vernetzung der Fasern besteht bevorzugt aus einer zufällig überkreuzten Anordnung der Fasern, die sich so bevorzugt ineinander verhaken. Besonders bevorzugt besteht die Vernetzung durch Klebstofftröpfchen an den Kreuzungspunkten der Fasern. Vernetzte Fasern können beispielsweise Gewebe, dünnlagige Watte, feine Lagen Krepppapier, Vliespapier o.Ä. sein.

Bevorzugt bilden die miteinander vernetzten Fasern ein Vlies, in welches sich die in der luftdichten Hülle befindlichen anderen Elemente bevorzugt mischen. Bevorzugt füllt dieses Gemisch den Hohlraum der luftdichten Hülle aus. Die vernetzten Fasern bilden bevorzugt mindestens eine Schicht innerhalb der luftdichten Hülle. Bevorzugt sind auch die im Wesentlichen inkompressiblen Elemente in mindestens einer Schicht in der luftdichten Hülle angeordnet.

In einem weiteren Ausführungsbeispiel der vorliegenden Erfindung ist eine Blutdruckmessvorrichtung (20) vorgesehen, bei der die luftdichte Hülle (32) Styroporkugeln (33) und vernetzte Fasern (34) aufweist.

Die Styroporkugeln bestehen bevorzugt aus einem Material mit hohem Oberflächenreibungskoeffizienten, besonders bevorzugt aus Styropor. Sie weisen einen Radius R auf. Der Radius variiert bzw. liegt bevorzugt zwischen 0,01 mm bis 10 mm, besonders bevorzugt zwischen 0,15 mm bis 4 mm.

Bevorzugt weist die luftdichte Hülle vernetzte Fasern auf.

Bevorzugt durch das Evakuieren der luftdichten Hülle legen sich die Styroporkugeln eng aneinander und verkeilen sich durch Reibung. Der verkeilte Verbund von Styroporkugeln und vernetzten Fasern ist bevorzugt steif und versteift somit das flexible Element.

Besonders bevorzugt ist ein flexibles Element, das eine Versteifungseinrichtung aufweist, eine mit Styroporkugeln und bevorzugt vernetzten Fasern gefüllte, luftdichte Hülle. Dadurch ist das Prinzip einer Vakuumschiene oder Vakuummatratze realisiert.

In einem weiteren Ausführungsbeispiel weist die Versteifungseinrichtung (31) einen Stapel von Blättern aus Papier (311) oder aus einem ähnlichen Material auf.

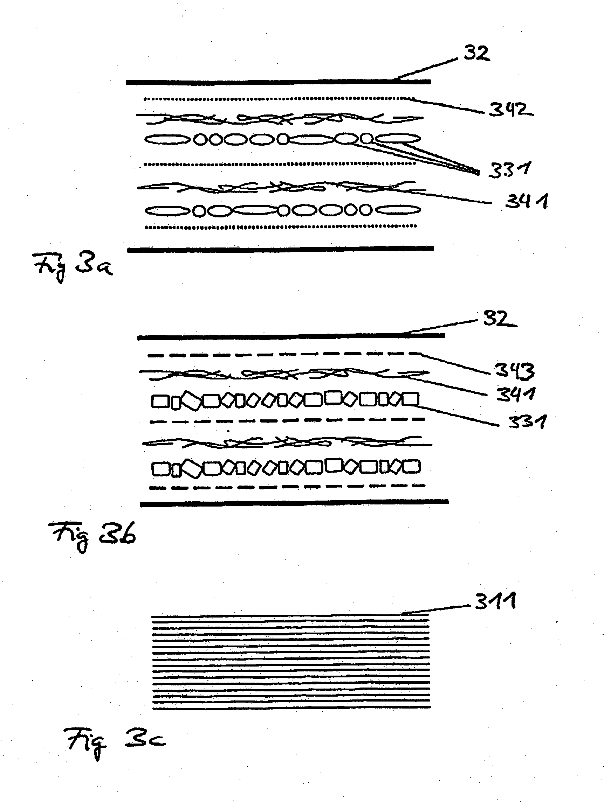

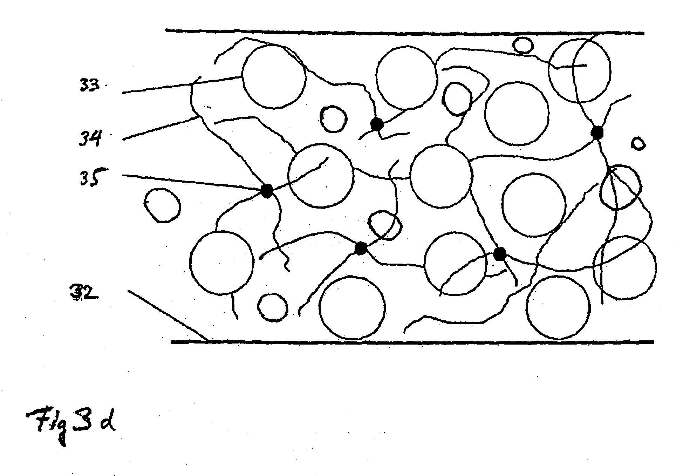

Dieser Stapel aus Papierlagen liegt bevorzugt in der luftdichten Hülle. Dadurch dass die Lagen Papier nahezu ohne Zwischenraum aufeinanderliegen (beispielsweise gestapelt wie zuvor beschrieben), ist die luftdichte Hülle bereits ohne eine Evakuierung sehr druckfest und bietet gute Eigenschaften für eine Blutdruckmessung. Wird nun die luftdichte Hülle zusätzlich noch evakuiert, bilden die Lagen Papier einen festen Verbund dessen Steifigkeit weiter zunimmt. (Abb.3: Sample 3). So wird ein sehr günstiges Signal-Rausch-Verhältnis erreicht.

In einem weiteren Ausführungsbeispiel der vorliegenden Erfindung ist eine Blutdruckmessvorrichtung (20) vorgesehen, bei der das flexible Element (30) mindestens einen Raststreifen (51) aufweist.

Der Raststreifen weist bevorzugt auf mindestens einer Seite ein bevorzugt sägezahnartiges Oberflächenprofil auf. Das Sägezahprofil ist bevorzugt derart gestaltet, dass sich zwei Raststreifen bei gegengleichem Übereinanderlegen der Raststreifen ineinander verkeilen, wenn sie in einer Richtung quer zum Sägezahnprofil verschoben werden, bis sie durch die Verkeilung nicht mehr verschoben werden können. Bevorzugt verkeilen sie sich jedoch nicht, wenn sie in der gegensätzlichen Richtung verschoben werden.

Bevorzugt weist die Blutdruckmessvorrichtung einen inneren und einen äußeren Raststreifen auf. Dies ist eine Art der äußeren Fixierung, welche zusätzlich oder anstatt einer vorhandenen äußeren Fixierung vorgesehen sein kann. Der innere Raststreifen ist bevorzugt auf der Innenseite des flexiblen Elements angebracht, so dass die Oberfläche mit dem Sägezahnprofil dem Körperteil zugewandt ist. Der äußere Raststreifen ist an der körperabgewandten Seite des flexiblen Elements bevorzugt an der äußeren Fixierung angebracht, so dass dessen Sägezahnprofil bevorzugt körperabgewandt ist. Der innere Raststreifen weist bevorzugt eine Öse auf, an welcher bevorzugt ein Zugband zum Einhängen vorgesehen ist, das durch eine bevorzugt am äußeren Raststreifen angebrachte Lasche durchführbar ist. Damit lassen sich nach dem Anlegen des flexiblen Elements um ein Körperteil die beiden Raststreifen gegeneinander durch Zug am Zugband fixieren. Besonders bevorzugt ist der innere Raststreifen direkt durch die Lasche am äußeren Raststreifen durchführbar. Die Rastreifen sind so durch Zug am Ende des inneren Raststreifens gegeneinander fixierbar. Durch die bevorzugte Kombination von Zugband und Raststreifen lässt sich das flexible Element an das Körperteil drücken. Die Raststreifen wirken als eine äußere Fixierung. Bevorzugt nachdem das flexible Element versteift wurde sind die Raststreifen gegeneinander unbeweglich. Die Raststreifen können bevorzugt auch als Ersatz für die konventionelle Blutdruckmanschette vorgesehen sein, falls auf diese verzichtet werden soll.

In einem weiteren Ausführungsbeispiel der vorliegenden Erfindung ist eine Blutdruckmessvorrichtung (20) vorgesehen, bei der eine Steuereinrichtung (60) zur Steuerung der Versteifungseinrichtung (31) vorgesehen ist.

Die Steuereinrichtung umfasst bevorzugt eine Pumpe und bevorzugt weitere Elemente, wie zum Beispiel eine elektronische Recheneinheit und/ oder steuerbare Ventile, um die Versteifungseinrichtung manuell oder elektronisch zu steuern. Die Steuereinrichtung ist bevorzugt über die vorhandenen Steuerleitungen mit den weiteren Elementen und/ oder den Komponenten der Blutdruckmessvorrichtung verbunden. Bevorzugt ist die Steuereinrichtung in einem Gehäuse untergebracht, das bevorzugt an dem flexiblen Element, besonders bevorzugt an der äußeren Fixierung angebracht ist. Bevorzugt ist die Steuereinrichtung dazu ausgelegt, die Versteifungseinrichtung bezüglich definierter Sollwerte zu regeln, wie zum Beispiel bezüglich der Güte des Vakuums. Besonders bevorzugt ist die Steuereinrichtung bevorzugt über eine Regelungseinrichtung zur Regelung der äußeren Fixierung oder bevorzugt der Blutdruckmanschette bezüglich definierter Sollwerte - wie zum Beispiel des Fixierungsgrads - eingerichtet.

In einem weiteren Ausführungsbeispiel der vorliegenden Erfindung ist eine Blutdruckmessvorrichtung (20) vorgesehen, bei der eine Analyseeinrichtung zur Analyse und/ oder Anzeige und/ oder Speicherung von Messdaten vorgesehen ist.

Die Analyseeinrichtung kann bevorzugt zusammen mit der Steuereinrichtung in einem Gehäuse untergebracht sein. Sie verfügt bevorzugt über eine Recheneinheit, welche bevorzugt dazu vorgesehen ist, die gemessenen Daten bezüglich messungsrelevanter Parameter, wie die Pulskonturparameter Schlagvolumen, maximale Druckanstiegsgeschwindigkeit und insbesondere HLI-Parameter (Pulsdruck-Variation PPV, Schlagvolumen-Variation SVV), zu analysieren. Besonders bevorzugt wird hierfür auf die Recheneinheit der Steuereinheit zurückgegriffen, so dass dann für die Analyseeinheit keine eigene Recheneinheit vorgesehen sein muss. Bevorzugt ist eine Anzeige, wie zum Beispiel ein kompaktes LCD oder OLED, vorgesehen. Sie erlaubt bevorzugt eine Visualisierung von bevorzugt berechneten Parametern, besonders bevorzugt von Zustandsgrößen, ganz besonders bevorzugt von Messwerten. Zudem können in der Analyseeinrichtung die Daten bevorzugt gespeichert werden und der Verlauf der Messung bevorzugt protokolliert werden. Die Analyseeinrichtung ist bevorzugt so vorgesehen, dass später messungsrelevante Informationen an einem Computer ausgelesen werden können.

Die Aufgabe wird weiterhin gelöst durch ein erfindungsgemäßes Verfahren zur Blutdruckmessung eines Lebewesens umfassend die Schritte:

- Anlegen eines flexiblen Elements (30) mit einem Drucksensorelement (40) an ein für die Messung relevantes Körperteil (10), so dass das flexible Element (30) eine körperteilangepasste Form einnimmt,

- Versteifen des flexiblen Elements (30) in der körperteilangepassten Form,

- Messung des Drucksignals über einen bestimmten Zeitraum während sich das flexible Element (30) in dem versteiften Zustand befindet,

- Überführung des Zustands des flexiblen Elements (30) zurück in den flexiblen Zustand.

Mit diesem Verfahren können die Pulskonturparameter und die dynamischen Parameter der Herz-Lungen-Interaktion (HLI) wie beispielsweise PPV, SVV, PEPV sowie weitere auf der Herz-Lungen-Interaktion beruhende abgeleitete Größen bestimmt werden, ohne dass eine aufwendige Kanülierung eines arteriellen Gefäßes erforderlich wäre. Dadurch können diese Parameter nicht-invasiv bestimmt werden. Bei einer entsprechenden Auswertung der pulsatilen Signale kann davon ausgegangen werden, dass die so genau gemessenen pulsatilen Signale direkt dem arteriellen Druck entsprechen. Bevorzugt wird für diese Auswertung mindestens einmal der systolische und diastolische Blutdruck über eine oszillometrische oder auskultatorische Methode bestimmt und die systolischen und diastolischen Werte des gemessenen pulsatilen Signals auf die zuvor bestimmten Werte geeicht.

Bevorzugt wird dieses Verfahren bei einem beatmeten, insbesondere bei einem kontrolliert beatmeten Patienten eingesetzt. Bei diesen kontrolliert beatmeten Patienten können diese Parameter wichtige Informationen liefern, da hier aufgrund des aufgewandten Drucks auf Lunge und mittelbar die Gefäße sowie das Herz des Patienten Volumenverschiebungen geschaffen werden.

Das Anlegen des flexiblen Elements erfolgt bevorzugt durch manuelles Andrücken des flexiblen Elements an das Körperteil. Durch die Verformbarkeit des flexiblen Elements lässt sich dieses bevorzugt schon zuvor in eine geeignete Form bringen, besonders bevorzugt ist das flexible Element bereits vorgeformt ausgeführt. Beim Anlegen passt sich das flexible Element dem Körperteil derart an, dass die Oberfläche des flexiblen Elements zu einem inversen Abbild des Oberflächenprofils des Körperteils wird. Bevorzugt bleiben keine Luftzwischenräume zwischen der dem Körperteil zugewandten Oberfläche des flexiblen Elements und dem Körperteil. Bevorzugt wird das flexible Element so angelegt, dass das Drucksensorelement über einer in dem Körperteil verlaufenden Arterie liegt. Beispielsweise würde bei Messung an der Arteria brachialis des Oberarms das Drucksensorelement bevorzugt an der Innenseite des Oberarms anliegen. Das Anlegen erfolgt so, dass das flexible Element das Körperteil bevorzugt zumindest teilweise bedeckt. Besonders bevorzugt bedeckt das flexible Element das Körperteil mit der gesamten verfügbaren Fläche. Bevorzugt umschließt das flexible Element das Körperteil. Bevorzugt wird das flexible Element über das Körperteil gestülpt. Besonders bevorzugt wird das flexible Element um das Körperteil gewickelt. Beim Anlegen des flexiblen Elements and das Körperteil wird bevorzugt der Druck, mit dem das flexible Element auf dem Körperteil aufliegt, durch ein Drucksensorelement gemessen, so dass eine Information über den Grad der Fixierung vorliegt. Besonders bevorzugt ist das hierfür verwendete Drucksensorelement ein schon an dem flexiblen Element angebrachtes Drucksensorelement. Bevorzugt entsteht durch das Anlegen ein Druck, der 10% des diastolischen Blutdrucks nicht übersteigt.

Das flexible Element wird an ein Körperteil eines Lebewesens, bevorzugt eines Menschen angelegt. Für eine Blutdruckmessung sind bevorzugt der Oberarm, besonders bevorzugt das Handgelenk eines Menschen geeignet. Bei anderen Lebewesen sind äquivalente Körperteile oder bevorzugt der Schwanz geeignet.

Das Versteifen erfolgt durch Aktivierung der Versteifungseinrichtung. Durch das Versteifen des flexiblen Elements lässt dieses sich nicht mehr verformen und erstarrt in der körperteilangepassten Form. Bevorzugt wird das flexible Element durch das Versteifen sowohl plastisch als auch elastisch unformbar. Besonders bevorzugt wird das flexible Element durch das Versteifen inkompressibel. Ganz bevorzugt wird das flexible Element durch das Versteifen nicht mehr verwindbar oder verbiegbar. Durch das Versteifen des flexiblen Elements entsteht eine rigide Ankopplung der an dem flexiblen Element angebrachten Drucksensorelemente an das Körperteil. Durch das Versteifen wird eine hydraulisch optimierte Kopplung mindestens eines an der Blutdruckmessvorrichtung angebrachten Sensors an das Körperteil erreicht.

Das Messen pulsatiler Signale findet dann über die Zeit statt. Die durch den Puls verursachten Druckschwankungen werden auf die an dem flexiblen Element angebrachten Drucksensorelemente übertragen und können dort abgegriffen werden. Diese Signale sind gegenüber den bei einer invasiven Messung des arteriellen Blutdrucks abgegriffenen Blutdrucksignalen stark gedämpft, da sie mittelbar über das Gewebe und das Drucksensorelement gemessen werden. Diese Signale werden damit bevorzugt von außen mittelbar abgegriffen. Dies geschieht bevorzugt über die Zeit, so dass eine Reihe von Messwerten zu speziellen Zeitpunkten vorliegt. Im versteiften Zustand des flexiblen Elements ist die Ankopplung des Drucksensorelements hydraulisch vorteilhafter als im flexiblen Zustand, weshalb die Messung vorzugsweise dann stattfindet, während sich das flexible Element in seinem steifen Zustand befindet.

Die Messung des Drucksignals erfolgt bevorzugt über einen bestimmten Zeitraum. Der Messzeitraum umfasst dabei bevorzugt mindestens einen respiratorischen Zyklus bzw. Atemzyklus, vorzugsweise mehrere, besonders bevorzugt drei oder mehr respiratorische Zyklen. Dies kann beispielsweise dadurch realisiert werden, dass der Druck, der von der äußeren Fixierung, bevorzugt von dem versteiften flexiblen Element, auf das Körperteil ausgeübt wird, über einen längeren Zeitraum innerhalb des pulsatilen Bereichs gehalten oder verlangsamt abgelassen wird. Im Gegensatz zur oszillometrischen Blutdruckmessung, bei der im Wesentlichen der mittlere Druck in der Manschette zum Zeitpunkt des Beginns der Pulsatilität, zum Zeitpunkt der maximalen Schwankungen bzw. zum Zeitpunkt des Verschwindens der Pulsatilität maßgeblich ist, werden bei dem HLI-Verfahren gemäß der vorliegenden Erfindung bevorzugt die arteriellen Pulsationen bevorzugt durch vorbekannte systolische und diastolische Werte kalibriert und dann mit genauer Formanalyse selbst ausgewertet.

Hierbei wird der Atemzyklus bevorzugt aus dem zeitlichen Verlauf der pulsatilen Schwankungen bestimmt. Hilfsweise, kann die Identifikation eines Atemzyklus aber auch über andere Messmethoden, zum Beispiel aus dem thorakalen elektrischen Impedanzsignal, das über EKG-Elektroden erfasst werden kann, vorgenommen werden. Bevorzugte weitere Verfahren zur Bestimmung des Atemzyklus sind beispielsweise die in der EP 01 813 187 beschriebenen. Hier werden auch weitere vorteilhafte Auswertungsmöglichkeiten für die erfindungsgemäß gewonnenen Blutdruckdaten angegeben, auf die hiermit Bezug genommen wird. Beispielsweise kann man bevorzugt dann eine Anzeige bestimmter Parameter unterdrücken, wenn zum Beispiel eine Arrhythmie order eine irreguläre Atmung (eben keine kontrollierte Beatmung) vorliegt.

Da das flexible Element von dem steifen Zustand wieder zurück in den flexiblen Zustand überführt werden kann, ist das flexible Element und auch die gesamte Blutdruckmessvorrichtung bevorzugt wiederverwendbar.

Bei einem weiteren erfindungsgemäßen Verfahren wird eine äußere Fixierung (50) über das flexible Element (30) angebracht.

Vorzugsweise erfolgt dies nach dem Anlegen des flexiblen Elements. Die Anbringung erfolgt vorzugsweise so, dass die äußere Fixierung eine Kraft bevorzugt gleichmäßig entlang der Auflagefläche von Körperteil und flexiblem Element auf das flexible Element aufbringt, so dass das flexible Element an das Körperteil gedrückt wird und bevorzugt das flexible Element an das Körperteil fixiert wird. Die an dem flexiblen Element angebrachten Drucksensorelemente oder weiteren Sensoren werden durch die äußere Fixierung an das Körperteil gedrückt. So werden das flexible Element und die auf der dem Körperteil zugewandten Innenseite des flexiblen Elements angebrachten Drucksensorelemente hydraulisch mit dem Körpergewebe gekoppelt. Bevorzugt wird die äußere Fixierung eingestellt, bevorzugt so, dass ein vorbestimmter Fixierungsgrad entsteht.

Der Fixierungsgrad steht in Relation zur Kraft, welche die äußere Fixierung auf das flexible Elements ausübt. Sie kann durch Einstellen der äußeren Fixierung variiert werden. So kann ein sicheres aber nicht zu festes Anliegen des flexiblen Elements gewährleistet werden. Beim Einstellen der äußeren Fixierung wird bevorzugt der Druck, mit dem das flexible Element auf dem Körperteil aufliegt, durch ein Drucksensorelement gemessen, so dass eine Information über den Grad der Fixierung vorliegt. Besonders bevorzugt ist das hierfür verwendete Drucksensorelement ein schon an dem flexiblen Element angebrachtes Drucksensorelement.

Die Fixierung erfolgt bevorzugt durch ein Gurtsystem. Dabei wird mindestens ein Gurt um das flexible Element und das Körperteil gewickelt und bevorzugt durch eine Schnalle gezogen und fixiert. Der Gurt kann auch durch eine Kombination von einer an den Gurt angebrachten Öse und einem Klettverschluss fixiert werden. Bevorzugt ist der Gurt einseitig fest mit dem flexiblen Element verbunden.

Besonders bevorzugt erfolgt die Fixierung durch mindestens einen Raststreifen. Besonders bevorzugt erfolgt die Fixierung durch einen inneren und einen äußeren Raststreifen, die vorrichtungsgemäß angebracht sind. In diesem Fall wird das flexible Element derart an das Körperteil angelegt, dass der innere Raststreifen auf dem äußeren Raststreifen zu liegen kommt. Die Raststreifen können gegeneinander verschoben werden, so dass die Fixierung des flexiblen Elements stärker wird. Die entgegengesetzte Verschiebung bewirkt ein Verkeilen der Raststreifen, so dass diese Verschiebung nur über die Länge maximal eines Sägezahns möglich ist. Das Sägezahnprofil der Raststreifen verhindert, dass sich die Fixierung automatisch wieder löst. Die Verschiebung der Raststreifen erfolgt vorzugsweise durch Zug an einem Zugband, das die beiden Enden der Raststreifen miteinander verbindet. Bevorzugt wird das flexible Element mit dem Zugband zunächst locker geschlossen. Der Zug an dem Zugband zur Fixierung des flexiblen Elements kann bevorzugt automatisch erfolgen, beispielsweise über einen mit Druckluft betriebenen Balg. Besonders bevorzugt kann aber auch auf das Zugband verzichtet werden und der innere Raststreifen direkt durch die Lasche des äußeren Raststreifens geführt werden. Dann ist es möglich, durch Zug an dem bereits durch die Lasche durchgeführten Ende des inneren Raststreifens die Raststreifen gegeneinander zu verschieben.

Ganz besonders bevorzugt erfolgt die äußere Fixierung durch ein elastisches Element, das mit Flüssigkeit oder Gas befüllt wird. Das elastische Element wird eng an das flexible Element fixiert. Bevorzugt ist es bereits an der Außenseite des flexiblen Elements fixiert. Durch Befüllen des elastischen Elements mit Flüssigkeit oder Gas wird das flexible Element und das Drucksensorelement eng an das Körperteil gepresst. Das elastische Element dehnt sich durch das Befüllen aus und drückt so das flexible Element sowie angebrachte Drucksensorelemente auf das Körperteil. Das Befüllen des elastischen Elements erfolgt vorzugsweise über einen oder mehrere Anschlussschläuche.

Bei einem weiteren bevorzugten Verfahren wird das flexible Element (30) inkompressibel versteift.

Die inkompressible Versteifung bewirkt, dass sich das flexible Element nicht mehr eindrücken lässt, bevorzugt in den Bereichen, in denen ein Drucksensorelement an dem flexiblem Element angebracht ist. Besonders bevorzugt lässt sich das flexible Element nicht in einem zirkulären Bereich um das Körperteil - beispielsweise eine Extremität - eindrücken, welches von der Blutdruckmessvorrichtung umschlossen wird. Dadurch können Druckschwingungen, die auf das flexible Element wirken, nicht in das Material des flexiblen Elements übertragen werden. Eine auf das inkompressibel versteifte Element wirkende Druckschwingung, führt nicht zur Verformung des flexiblen Elements und somit nicht zur Dämpfung der Druckschwingung aufgrund von Energieabsorbtion im flexiblen Element durch Verformung.

Bei einem weiteren bevorzugten Verfahren wird das flexible Element (30) durch Evakuieren der darin befindlichen Luft versteift.

Das Aktivieren der Versteifungseinrichtung erfolgt bevorzugt durch Abpumpen bzw. Evakuieren der Luft in der Versteifungseinrichtung, sofern die Versteifungseinrichtung eine luftdichte Hülle aufweist. Es kann bevorzugt auch ein Vakuum angelegt werden. Dabei wird die Luft innerhalb der luftdichten Hülle über mindestens einen Anschlussschlauch abgesaugt. Es wird solange Luft aus der luftdichten Hülle abgesaugt, bis ein Vakuum innerhalb der luftdichten Hülle entsteht. Das Absaugen erfolgt bevorzugt über eine an dem Anschlussschlauchende angebrachte Pumpe. Die luftdichte Hülle befindet sich innerhalb des flexiblen Elements, so dass die Versteifungseinrichtung allgemein durch Evakuieren der in dem flexiblen Element befindlichen Luft aktiviert wird.

Bei einem weiteren bevorzugten Verfahren wird das flexible Element (30) durch Befüllen mit Druckluft versteift.

Bevorzugt wird bei Verwendung von zwei miteinander verschweißten Lagen sehr dehnungssteifen Materials (z.B. gummiertes Gewebe ähnlich zu einem Fahrrad- oder Autoreifen) als luftdichte Hülle das flexible Element durch Befüllen mit Druckluft versteift. Die Hülle wird durch das dehnungssteife Material sofort hart. Für optimale Messergebnisse ist bevorzugt ein Druck nahe dem arteriellen Mitteldruck einzustellen. Dafür wird mindestens eine Kammer der luftdichten Hülle aufgeblasen. Der erforderliche Druck zum Verhärten des flexiblen Elements übersteigt den Systolendruck so weit, dass dadurch der Systolendruck die luftdichte Hülle nicht verformen kann und damit auch keine Störungen oder Dämpfungen auftreten können. Durch weiteres Aufblasen mit hohem Druck lässt sich die innere Lage der luftdichten Hülle geringfügig dehnen und damit der auf den Arm des Patienten wirkende Druck erhöhen. So kann auf den arteriellen Mitteldruck (Messdruck) geregelt werden.

Bei einem weiteren bevorzugten Verfahren wird eine konventionelle Blutdruckmanschette als äußere Fixierung (50) verwendet.

Eine konventionelle Blutdruckmanschette ist aus dem Stand der Technik hinreichend bekannt. Sie kann an ein Körperteil, bevorzugt an den Oberarm oder das Handgelenk angelegt werden, wobei das Anlegen bevorzugt ein Umwickeln der Manschette um oder das Überstülpen der Manschette über das Körperteil bezeichnet. Sie wird dann in dieser Position mit Hilfe von angebrachten Klett- und/oder Gurtverschlüssen an das Körperteil fixiert. Durch Befüllen der Manschette mit Luft oder einer Flüssigkeit erhöht sich Ihr Volumen und sie drückt auf das Körperteil. Durch Ablassen von Füllmaterial kann der ausgeübte Druck erniedrigt werden. So ist es bevorzugt auch möglich, nicht das Volumen, sondern den Druck in der Manschette so einzustellen, dass durch eine Kompression des jeweiligen Körperteils eine indirekte Ankopplung der an dem flexiblen Element angebrachten Drucksensorelemente an die Volumenschwankungen der arteriellen Blutgefäße erfolgt.

Eine solche Blutdruckmanschette wird bevorzugt um das flexible Element gelegt, besonders bevorzugt über das flexible Element gestülpt. Sie bedeckt dann das flexible Element zumindest teilweise. Durch Befüllen der konventionellen Blutdruckmanschette übt die Blutdruckmanschette Druck auf das flexible Element aus, das dann folglich mitsamt der an ihr angebrachten Drucksensorelemente an das Körperteil gepresst wird und das Körperteil zusammendrückt.

Bei einem weiteren bevorzugten Verfahren wird beim Versteifen des flexiblen Elements (30) die evakuierte Luft in die äußere Blutdruckmanschette gepumpt.

Bevorzugt besteht mindestens ein Verbindungsschlauch mit bevorzugt mindestens einem Ventil zwischen der konventionellen Blutdruckmanschette und dem flexiblen Element. Besonders bevorzugt ist eine Pumpe zusätzlich zu dem Verbindungsschlauch zwischen dem flexiblen Element und der konventionellen Blutdruckmanschette vorgesehen. Luft aus dem flexiblen Element kann nun in die Blutdruckmanschette gepumpt werden. So wird das flexible Element entleert und damit steif und gleichzeitig wird die Blutdruckmanschette mit Luft befüllt und drückt das flexible Element auf das Körperteil.

Dieses bevorzugte Verfahren hat zwei Vorteile. Erstens kann mit nur einem Aktuator, bevorzugt der Pumpe, sowohl das flexible Element als auch die äußere Fixierung, bevorzugt die konventionelle Blutdruckmanschette, gesteuert werden. Zweitens erfolgt somit das Abpumpen des flexiblen Elements gleichzeitig mit dem Aufpumpen der Blutdruckmanschette, so dass die Ausführungszeit dieser beiden Prozesse in der Summe minimiert ist.

Bei einem weiteren bevorzugten Verfahren wird der systolische, diastolische und mittlere Blutdruck durch Variation des Drucks in der Blutdruckmanschette gemessen.

Der Druck, den die Blutdruckmanschette auf das flexible Element ausübt, kann, wie wenn das flexible Element nicht vorhanden wäre, dazu genutzt werden, mittels bekanntem oszillatorischen Messprinzip systolischen, diastolischen und mittleren Blutdruck zu messen. Hierfür wird Luft oder eine Flüssigkeit in die konventionelle Blutdruckmanschette gepumpt oder abgelassen. Bevorzugt befindet sich das flexible Element dabei nicht in seinem versteiften Zustand. Die pulsatilen Signale werden bevorzugt über eines der an dem flexiblen Element angebrachten Drucksensorelemente gemessen. Besonders bevorzugt wird die Messung in Intervallen durchgeführt, die jeweils aus der Variation des Fixierungsgrads der äußeren Fixierung, bevorzugt des Drucks in der konventionellen Manschette, mit anschließendem Versteifen des flexiblen Elements, gefolgt von der Messung pulsatiler Signale durch einen der Drucksensorelemente mit abschließender Überführung des flexiblen Elements zurück in seinen flexiblen Zustand bestehen.

Die oszillometrische Blutdruckmessung des Standes der Technik beruht grundsätzlich darauf, dass bei einer von außen anliegenden Druckmanschette die arteriellen Blutgefäße Kaliberschwankungen aufweisen, solange der Manschettendruck geringer als der systolische und größer als der diastolische Blutdruck ist. Diese Kaliberschwankungen der arteriellen Blutgefäße führen wiederum zu pulsatilen Druckschwankungen in der Blutdruckmanschette. Bei einem Manschettendruck, der größer als der systolische Blutdruck ist, werden die arteriellen Blutgefäße während des gesamten Herzzyklus vollständig komprimiert und es treten somit keine Kaliberschwankungen der Gefäße und keine pulsatilen Druckschwankungen in der Manschette auf. Unterschreitet der Manschettendruck den diastolischen Blutdruck, so sind die arteriellen Blutgefäße während des gesamten Herzzyklus vollständig geöffnet und es treten ebenfalls keine pulsatilen Schwankungen auf. Das eigentliche Messprinzip der oszillometrischen Blutdruckmessung ist nunmehr, dass der Druck in der Manschette solange erhöht wird, bis keine pulsatilen Druckschwankungen mehr auftreten. Sodann wird der Druck meist kontinuierlich reduziert und es werden hierbei die Druckwerte in der Manschette identifiziert, bei denen die Pulsatilität beginnt, maximal ist, bzw. verschwindet. Aus diesen Kennwerten werden der systolische, mittlere und diastolische Blutdruck bestimmt.

Bei einem weiteren bevorzugten Verfahren wird das flexible Element (30) bei einem arteriellen Druck zwischen dem Mitteldruck und dem diastolischen Druck versteift.

Bevorzugt wird der Druck der äußeren Fixierung so eingestellt, dass die äußere Fixierung einen Druck zwischen dem systolischen und dem diastolischen Druck im pulsatilen Bereich, bevorzugt einen Druck im pulsatilen Bereich, bei dem die größten arteriellen Oszillationen auftreten, ausübt. In diesem Bereich ist die Amplitude der pulsatilen Signale am höchsten und damit am deutlichsten zu detektieren.

Bevorzugt kann hierzu der Druck der äußeren Fixierung auf das flexible Element und damit auf das Körperteil von null beginnend erhöht werden, bis die ersten pulsatilen Signale wahrgenommen werden können - dieser dann herrschende Druck entspricht grob dem diastolischen Druck. Wenn nun weiter der Druck erhöht wird, dann gibt es einen zweiten Zeitpunkt, zu dem keine pulsatile Signale mehr gemessen werden können - dies entspricht dem systolischen Druck. Diese Werte können auch in der anderen Richtung ermittelt werden, d.h. von einem überhöhten Druck kommend kann festgestellt werden, wann ein erstes pulsatiles Signal empfangen wird (systolischer Druck) und ab wann bei weiterer Druckminderung kein Signal mehr empfangen wird (diastolischer Druck). Wenn nun ein Wert zwischen diesen beiden zu diesen Zeitpunkten applizierten Drücken verwendet wird, so befindet man sich im pulsatilen Bereich. Die Amplituden sind umso größer, je näher man in der Mitte dieses Bereichs misst, bevorzugt also im Mittelwert zwischen den beiden Drücken, d.h. dem Mitteldruck. Besonders bevorzugt wird nahe unterhalb des Mitteldrucks gemessen. Ganz besonders bevorzugt wird der Druck, bei dem gemessen wird ($P_{mess}$), über die Formel

$$P_{mess} = P_{dias} + 1/3 \ (P_{sys} - P_{dias})$$

abgeschätzt, wobei $P_{sys}$ der systolische und $P_{dias}$ der diastolische Blutdruck ist. Idealerweise wird $P_{mess}$ aber nach Kalibrierung des pulsatilen Signals durch Integration über die Zeit ermittelt.

In diesem Bereich können die maximalen Amplituden der pulsatilen Signale erwartet werden und damit die am besten auszuwertenden Signale.

Bei einem Druck in der konventionellen Manschette zwischen dem mittleren Blutdruck und dem diastolischen Blutdruck werden erfahrungsgemäß die größten Amplituden der pulsatilen Signale erwartet. Insbesondere treten die größten Amplituden erfahrungsgemäß nahe unterhalb des mittleren Blutdrucks auf. Durch das Versteifen des flexiblen Elements in diesem Druckbereich erfolgt die hydraulisch optimierte Ankopplung der an dem flexiblen Element angebrachten Drucksensorelemente in einem für die Messung der pulsatilen Signale optimalen Bereich. Bevorzugt werden bei einem in diesem Druckbereich versteiften flexiblen Element die pulsatilen Signale über die Zeit gemessen.

Bei einem weiteren bevorzugten Verfahren werden die Versteifung des flexiblen Elements (30) gesteuert und/ oder der Grad der Fixierung (50) durch eine Steuereinrichtung (60) gesteuert und/ oder die Sensorwerte erfasst, insbesondere auch analysiert und/ oder anzeigt und/ oder gespeichert.

Bevorzugt ist es möglich die Versteifung des flexiblen Elements konstant zu halten, bevorzugt über eine Regelung durch die Steuereinheit. Bei Verwendung einer Versteifungseinrichtung, die durch Anlegen eines Vakuums aktiviert wird, kann die Regelung der Versteifung bevorzugt bezüglich der Güte des Vakuums erfolgen. Besonders bevorzugt kann die Versteifung des flexiblen Elements gezielt variiert werden und es kann zwischen dem versteiften Zustand und dem flexiblen Zustand kontinuierlich gewechselt werden. Bevorzugt werden damit festgelegte Messabläufe ermöglicht. Diese Steuerung erfolgt bevorzugt durch die Steuereinheit und kann auch wiederum bezüglich bestimmter Messwerte geregelt werden, beispielsweise der Güte des Vakuums.

Bevorzugt ist es möglich den Grad der Fixierung, den die äußere Fixierung aufweist, konstant zu halten, bevorzugt über eine Regelung durch die Steuereinheit. Der Grad der Fixierung ist eine Funktion des Drucks, den die äußere Fixierung über das flexible Element auf das Körperteil ausübt. Bevorzugt wird daher der Grad der Fixierung aus diesem Druck hergeleitet, der bevorzugt durch ein Drucksensorelement gemessen wird. Besonders bevorzugt ist dieses Drucksensorelement ein bereits am flexiblen Element angebrachtes Drucksensorelement. Die Regelung des Grads der Fixierung erfolgt daher bevorzugt bezüglich des auf das Körperteil ausgeübten Drucks. Besonders bevorzugt kann der Grad der Fixierung gezielt variiert werden. Bevorzugt werden damit festgelegte Messabläufe ermöglicht. Die des Fixierungsgrads Steuerung erfolgt bevorzugt durch die Steuereinheit. Es kann bevorzugt auch eine Regelung des Fixierungsgrads bezüglich bestimmter Messwerte erfolgen, beispielsweise bezüglich des auf das Körperteil ausgeübten Drucks.

Die genannten Steuerungen und Regelungen können unabhängig voneinander, bevorzugt in geplanter Abhängigkeit zueinander erfolgen. Bevorzugt erfolgen sie zu bestimmten Zeitpunkten des Verfahrens, besonders bevorzugt während des gesamten Verfahrens.

Die Analyse erfolgt bevorzugt kontinuierlich mit neu eingehenden Messdaten, besonders bevorzugt zu einem oder mehreren Zeitpunkten. Bei der Analyse der gemessenen pulsatilen Signale werden bevorzugt die einzelnen Messwerte in Messwerte zusammengefasst, die einem Herzschlag zuzuordnen sind. Darüber hinaus kann auch eine Zuordnung zu einem respiratorischen Zyklus erfolgen. So können dann beispielsweise nach Entfernung von Artefakten Minimum und Maximum der einzelnen Blutdruckschwankungen pro Herzschlag ermittelt werden und die Schwankungen innerhalb mindestens eines Atemzyklus (Respirationszyklus) ermittelt werden.

Auf diese Weise ist es möglich, die gewünschten Parameter der Herz-Lungen-Interaktion (HLI) zu bestimmen.

Bei der nicht-invasiven Messung der HLI ist es gemäß der vorliegenden Erfindung bevorzugt vorgesehen, dass die systolischen und diastolischen Blutdruckwerte als Randwerte vorab bestimmt werden und darüber hinaus die Variation dieser Werte, die auf den respiratorischen HLI-Parametern beruhen.

Bevorzugt umfassen die HLI-Parameter die Schlagvolumen-Variation (SVV), die Pulsdruck-Variation (PPV) und/ oder die Präejektionsphasen-Variation (PEPV). Als HLI-Parameter können auch weitere auf der HLI beruhende abgeleitete Größen berechnet werden, wie z.B. die respiratorische Schwankungen der Pulswellengeschwindigkeit oder die respiratorische Variationsbreite der Druckanstiegsgeschwindigkeit.

Bevorzugt wird die respiratorische Variationsbreite der HLI ermittelt. Dabei werden bevorzugt die Maxima und die nachfolgenden Minima bestimmt- alternativ die Minima und die nachfolgenden Maxima, d.h. die Blutdruckamplitude wird aus dem systolischen und dem vorangehenden diastolischen Druck ermittelt. Anschließend wird die Amplituden-Variation über den respiratorischen Zyklus als Maß für die Pulsdruckvariation ermittelt. Grundsätzlich sind die an einem nicht-invasiven Drucksensorelement gemessenen Druckschwankungen, die durch die pulsatilen Kaliberschwankungen der Blutgefäße hervorgerufen werden, erheblich kleiner als die pulsatilen Druckschwankungen im arteriellen Blutgefäß. HLI-Parameter wie das PPV und das SVV sind jedoch Relativmaße (in der Regel werden sie in % angegeben) und die relative prozentuale Variation des in die Manschette fortgeleiteten Signals steht in engem Zusammenhang mit der respiratorischen Variation der HLI im arteriellen Blutgefäß. Gleiches gilt für die PEPV, bei der es sich jedoch um die Variationsbreite einer zeitlichen Dimension handelt. Bei dieser Ausprägung des HLI-Messverfahrens kann für die Erfassung der Verzögerungszeit zwischen elektrischer Aktivität und mechanischer Auswurfphase des Herzens zusätzlich beispielsweise ein Elektrokardiogramm für die zeitliche Erfassung des Beginns der elektrischen Herzaktivität verwendet werden. Die PEPV als HLI-Parameter kann alternativ auch aus der Zeitdifferenz zwischen einem elektrokardiographischen und einem photoplethysmographischen Signal erfasst werden.

Es ist bevorzugt auch möglich, die pulsatilen Signale mit einem Faktor zu multiplizieren bzw. eine Korrekturfunktion zu verwenden, um dadurch die auftretende Dämpfung der arteriellen Drucksignale zu kompensieren. Dieser Faktor kann bevorzugt empirisch durch statistische Erhebung an einem größeren Patientenkollektiv ermittelt werden. Besonders bevorzugt kann aus einer direkten invasiven und simultanen nicht-invasiven Messung der pulsatilen Signale und der Auswertung dieser Signale dann der Faktor der Dämpfung zurückgerechnet werden. Dieser Faktor kann dann bei den folgenden nicht-invasiven Messungen herangezogen werden, um die gemessenen pulsatilen Signale in die tatsächlichen aktuellen arteriellen Werte umzurechnen.

Die Dämpfung, die zwischen arteriellem "wahren Drucksignal" und dem Drucksignal am Drucksensorelement auftritt, ist im Wesentlichen eine Funktion der Kompressibilität des Gewebes. Diese Übertragungsfunktion kann sehr vereinfacht durch einen Faktor kompensiert werden. Grundsätzlich handelt es sich um eine Übertragungsfunktion die z.B. durch ein Ersatzschaltbild bestehend aus Reihen und Parallelschaltungen von Widerständen und Kapazitäten dargestellt werden kann, im einfachsten Fall der Parallelschaltung eines Widerstandes und eines Kondensators. Die numerische Kompensation dieser Übertragungsfunktion ist eine Dekonvolution. Bei Kenntnis der grundsätzlichen Charakteristik der arteriellen Druckkurve (z.B. auf der Basis einer idealisierte Modellkurve) und Kenntnis der grundsätzlichen Charakteristik der Übertragungsfunktion (z.B. Widerstand und Kapazität in Parallelschaltung) lassen sich die Parameter für die Übertragungsfunktion zur exakten Korrektur und Rückrechnung auf das "wahre intravasale Drucksignal" bevorzugt folgendermaßen ermitteln: In einem ersten Schritt wird mittels konventioneller oszillometrischer Druckmessung der systolische und der diastolische bzw. mittlere arterielle Druck ermittelt. Das flexible Element befindet sich dabei bevorzugt in seinem flexiblen Zustand. In einem zweiten Schritt wird der mittlere Druck in der Manschette bei demjenigen Druck "geclampt", bei dem die maximale pulsatile Signalqualität zu verzeichnen ist (in der Regel beim mittleren arteriellen Druck $P_{mess} = P_{dias} + 1/3 \, (P_{sys} - P_{dias})$ bzw. dem durch zeitliche Integration erhaltenen $P_{mess}$). Das flexible Element wird dann versteift, um eine hohe Signalqualität zu gewährleisten.

Auf diese Weise ist es möglich, die gemessenen pulsatilen Signale zu verwenden, um hiermit Pulskonturverfahren zur Abschätzung des Herzzeitvolumens (HZV) oder des Pulskonturschlagvolumens durchzuführen.

Bevorzugt werden Messwerte und analysierte Parameter angezeigt. Die Anzeige kann bevorzugt an einem Bildschirm und/oder durch Ausdruck an einem angeschlossenen Rechner erfolgen, besonders bevorzugt an einem an der Blutdruckmessvorrichtung angebrachten Anzeigegerät, beispielsweise ein LCD. Die Anzeige von Messwerten und analysierten Parametern ist kontinuierlich ab dem Zeitpunkt ihrer Verfügbarkeit möglich, erfolgt bevorzugt aber erst zu einem bestimmten Zeitpunkt, besonders bevorzugt zu mehreren Zeitpunkten.

Bevorzugt werden die für eine spätere Verarbeitung, Analyse, oder Archivierung benötigten Daten, die durch das Verfahren erhoben wurden, gespeichert. Hierzu weist die Steuereinheit bevorzugt mindestens ein Speicherelement auf, das beispielsweise durch einen angeschlossenen Rechner auslesbar ist. Die Speicherung dieser Daten ist kontinuierlich ab dem Zeitpunkt ihrer Verfügbarkeit möglich, erfolgt bevorzugt aber erst zu einem bestimmten Zeitpunkt, besonders bevorzugt zu mehreren Zeitpunkten.

Bei einem weiteren bevorzugten Verfahren wird zusätzlich die elektrische Impedanz des Körperteils (10) gemessen.

Bevorzugt wird zusätzlich zu den verfahrensgemäßen Messungen die elektrische Impedanz des Körperteils gemessen. Besonders bevorzugt kann aber auch ausschließlich eine Impedanzmessung des Körperteils erfolgen. Dabei werden mindestens eine Anregungs- und mindestens eine Detektionselektrode verwendet. Es können bevorzugt mehrere Anregungs- und Detektionselektroden verwendet werden, um die elektrische Impedanz an verschiedenen Stellen des Körperteils zu messen. Die Elektroden sind an der Blutdruckmessvorrichtung angebracht. Bevorzugt sind sie an dem flexiblen Element angebracht, so dass sie Kontakt zur Haut des Körperteils aufweisen. Dieser Kontakt kann bevorzugt durch leitendes Gel verbessert werden. Anregungs- und Detektionselektrode bilden durch den Widerstand der Haut, Unterhaut, Muskeln, Fett, Knochen, Blut etc. einen geschlossenen Stromkreis, in dem ein definierter, für den Körper ungefährlicher Strom, bevorzugt ein Wechselstrom fließt. Durch den bevorzugt separat gemessenen Spannungsabfall zwischen Anregungs- und Detektionselektrode bzw. den Injektionselektroden kann so der ohmsche Widerstand bzw. Wechselstromwiderstand berechnet werden, der durch das Körperteil, bevorzugt durch den Extremitätenzylinder gegeben ist. Dieser variiert mit den arteriellen Pulsationen und korreliert mit dem arteriellen Blutfluss in dem Körperteil, da sich die den Widerstand beeinflussenden Aderquerschnitte und die Menge des Blutes in den Adern mit den Pulsationen ändern. Insbesondere korreliert die Impedanz bzw. deren erste Ableitung mit der pulsatilen Querschnittsänderung der z.B. im Oberarm befindlichen Arterien, besonders der Arteria brachialis. Die Messung der Impedanz des Körperteils stellt daher weitere Informationen über die pulsatilen Signale zur Verfügung. Diese Informationen werden bevorzugt für eine exaktere Berechnung der HLI-Parameter mit den Messdaten der Drucksensorelemente kombiniert. Die Informationen aus der Impedanzmessung können besonders bevorzugt dazu verwendet werden, die Messdaten der an dem flexiblen Element angebrachten Drucksensorelemente korrigierend zu verändern und/ oder vice versa. Die Messung der Impedanz erfolgt bevorzugt kontinuierlich, besonders bevorzugt zu einem oder mehreren Zeitpunkten, besonders bevorzugt alternierend zur Messung der pulsatilen Signale durch ein Drucksensorelement.

Bei einem weiteren bevorzugten Verfahren wird während einer Messung und/ oder danach aus einem oder mehreren der gemessenen Druckverläufe die arterielle Kurvenform bestimmt. Hierbei werden bevorzugt übliche Signaltransformationen verwendet.

Bevorzugt werden aus den pulsatilen Signalen Werte für die Durchführung einer Pulskonturmethode abgeleitet. Für die Durchführung einer Pulskonturmethode werden die absoluten Blutdruckwerte benötigt. Aufgrund der gegenüber den Manschetten zur oszillometrischen Blutdruckessung des Standes der Technik verbesserten Signalqualität, ist eine Art nicht-invasive kontinuierliche Blutdruckmessung möglich, inkl. aller weiterer Analysemöglichkeiten wie z.B. Pulskonturverfahren.

Bei einem weiteren bevorzugten Verfahren wird nach einer bestimmten Dauer die Versteifung des flexiblen Elements (30) rückgängig gemacht.

Die bestimmte Dauer richtet sich vorrangig nach der Art und dem Ziel der Messungen, sowie der Zeitdauer, während der das flexible Element ohne Schädigung des Körperteils versteift sein darf. Vorzugsweise wird das flexible Element für eine Dauer von mindestens n Respirationszyklen versteift und danach wird die Versteifung wieder rückgängig gemacht, wobei n Element der reellen Zahlen ist, bevorzugt n=1, bevorzugt n=2, besonders bevorzugt n=3 oder größer. Das Rückgängigmachen der Versteifung erfolgt durch Deaktivierung der Versteifungseinrichtung, so dass das flexible Element wieder verformbar wird. Bevorzugt wird das flexible Element wieder plastisch oder elastisch verformbar. Bei Verwendung einer luftdichten Hülle als Versteifungseinrichtung wird die Versteifungseinrichtung durch Zuführen von Luft in die luftdichte Hülle bzw. durch Belüften der luftdichten Hülle deaktiviert. Das Belüften erfolgt über einen oder mehrere Anschlussschläuche. An diesem Anschlussschlauch ist bevorzugt eine Pumpe, besonders bevorzugt ein Druckbehälter, der genügend Druckluft enthält, angeschlossen, so dass das Belüften der luftdichten Hülle rasch erfolgt.

Bei einem weiteren bevorzugten Verfahren wird nach einer bestimmten Dauer die äußere Fixierung (50) gelockert.

Die bestimmte Dauer richtet sich vorrangig nach der Art und dem Ziel der Messungen, sowie der Zeitdauer, während der die äußere Fixierung ohne Schädigung des Körperteils Druck auf das flexible Element und somit auf das Körperteil ausüben darf. Bei zu langer Anwendung einer äußeren Fixierung wird Flüssigkeit aus dem Körpergewebe verdrängt. Vorzugsweise übt die äußere Fixierung für eine Dauer von mindestens k Respirationszyklen Druck aus und danach wird sie gelockert wobei k Element der reellen Zahlen ist, bevorzugt ist k=1, bevorzugt k=2, besonders bevorzugt k=3 oder größer.

Die Lockerung der äußeren Fixierung erfolgt durch Lockerung der Zugbänder, sofern sie vorhanden sind, Lockerung etwaig angebrachter Gurtsysteme, und Entleeren des elastischen Elements, bevorzugt Entleerung der konventionellen Blutdruckmanschette, jeweils sofern die entsprechende Fixierungsform vorhanden ist.

Die Erfindung soll nun anhand von Zeichnungen weiter veranschaulicht werden. Hierbei zeigen:

Fig. 1a    einen schematischen Querschnitt einer erfindungsgemäßen Blutdruckmessvorrichtung,

Fig. 1b    einen schematischen Querschnitt einer erfindungsgemäßen Blutdruckmessvorrichtung in ringförmiger Ausführung und mit einer Blutdruckmanschette als äußere Fixierung.

Fig. 2    Aufsicht auf ein Drucksensorelement, das als Gelkissen ausgeführt ist, wobei die Impedanzelektroden quer zur Longitudinalrichtung der Extremität zum Liegen kommen,

Fig. 3a    ein Schema einer bevorzugten Ausführungsform der Versteifungseinrichtung des flexiblen Elements in Form einer mit Reiskörnern und Stoffgewebe und Wollfasern gefüllten luftdichten Hülle,

Fig. 3b    ein Schema einer bevorzugten Ausführungsform der Versteifungseinrichtung des flexiblen Elements in Form einer mit Plastikgranulat, Kunststoffnetzen und Baumwollfasern gefüllten luftdichten Hülle,

Fig. 3c    ein Schema einer bevorzugten Ausführungsform der Versteifungseinrichtung des flexiblen Elements in Form eines Papierstapels,

Fig. 3d    ein Schema einer bevorzugten Ausführungsform der Versteifungseinrichtung des flexiblen Elements in Form einer mit Styroporkugeln und Fasern gefüllten luftdichten Hülle,

Fig. 4    eine dreidimensionale Zeichnung einer bevorzugten Ausführungsform der erfindungsgemäßen Blutdruckmessvorrichtung mit Blutdruckmanschette, Gelkissen und Raststreifen,

Fig. 5    ein schematisches Ablaufdiagramm erfindungsgemäßer Verfahren,

Fig. 6    den zeitlichen Verlauf ausgewählter Zustandsgrößen während einer beispielhaften Anwendung der Blutdruckmessvorrichtung,

Fig. 7    den zeitlichen Verlauf des durch einen Drucksensor gemessenen Druckverlaufs bei Ausführung der Blutdruckmessvorrichtung mit einer äußeren Fixierung, die das flexible Element nicht vollständig an allen Enden überlappt und

Fig. 8    den zeitlichen Verlauf des durch einen Drucksensor gemessenen Druckverlaufs bei Ausführung der Blutdruckmessvorrichtung mit einer äußeren Fixierung, die das flexible Element vollständig an allen Enden überlappt.

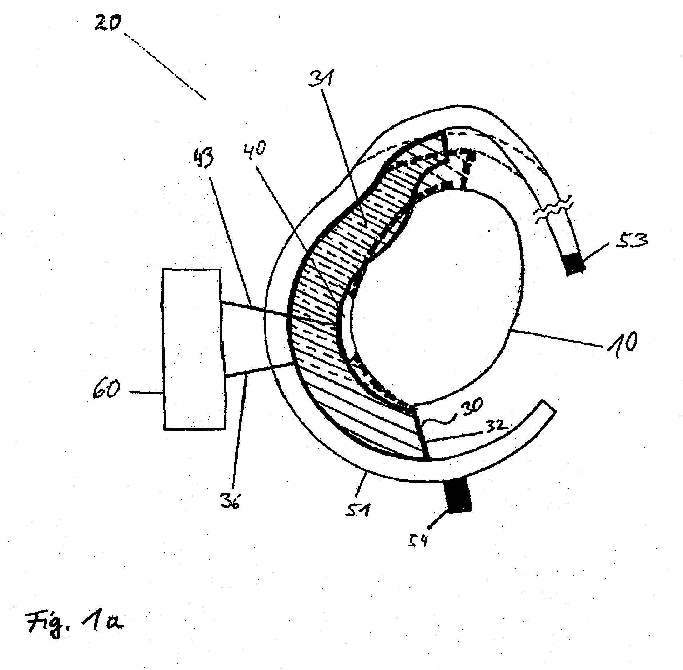

**Figur 1a** zeigt einen schematischen Querschnitt einer erfindungsgemäßen Blutdruckmessvorrichtung 20. Sie umfasst ein flexibles Element 30 mit Versteifungseinrichtung 31 und Steuerleitung 36, ein Drucksensorelement 40 mit Sensorkabel 43, einen Raststreifen 51, eine Öse 53, eine Lasche 54 und eine Steuereinrichtung 60.

Ein Körperteil 10 ist als Ellipse dargestellt. Das flexible Element 30 ist begrenzt durch eine luftdichte Hülle 32, welche durch eine dicke schwarze Umrandung dargestellt ist. Sie enthält ein Füllmaterial, bestehend aus Styroporkugeln und vernetzten Fasern. Dieses Füllmaterial ist durch eine Schraffur (von links oben, nach rechts unten) gekennzeichnet und gleichmäßig in der luftdichten Hülle 32 verteilt und dient als Versteifungseinrichtung 31. Die Formveränderbarkeit ist beispielhaft dadurch dargestellt, dass die Bereiche, die nach Anpassung an das Körperteil 10 ihre Form verändert haben, gestrichelt gezeichnet sind. Ein Anschlussschlauch 36 ist an der luftdichten Hülle 32 bzw. dem flexiblen Element 30 als Steuerleitung für das flexible Element 30 angebracht. Er ist als dicke Linie dargestellt. Das Drucksensorelement 40 ist an dem flexiblen Element angebracht. Es ist an der Oberfläche des flexiblen Elements 30 angebracht, die dem Körperteil 10 zugewandt ist, also der Innenseite. An dem Drucksensorelement ist ein Sensorkabel 43 angebracht. Es ist als dicke Linie gekennzeichnet. Der Raststreifen 51 ist um das flexible Element 30 angeordnet. In dem gezeigten Querschnitt überlappt der Raststreifen 51 das flexible Element 30 an zwei Seiten. Der Querschnitt zeigt eine Anbringungsvariante des Raststreifens an dem flexiblen Element 30. Der Raststreifen weist eine Öse 52 auf und eine Lasche 53 auf. Diese sind als kleine schwarze Vierecke dargestellt. Das Sensorkabel 43 des Drucksensorelements und der Anschlussschlauch 36 für das flexible Element 36 führen zu einer Steuereinrichtung 60. Die Steuereinrichtung ist als großes Rechteck dargestellt.

Beim Anlegen des flexiblen Elements 30 an das Körperteil 10 wird es durch Auflegen der Innenseite an das Körperteil 10 und Aufdrücken auf das Körperteil 10 in eine Form gebracht, die der Oberfläche des Körperteils angepasst ist. Die Innenseite des flexiblen Elements 30, erhält dadurch ein Oberflächenprofil, das an bevorzugt jedem Punkt der Oberfläche - bis auf die von einem Drucksensorelement bedeckten Flächen - in direktem Kontakt zu dem Körperteil 10 steht. Das Drucksensorelement 40 steht somit auch in direktem Kontakt zu dem Körperteil 10. Befindet sich das flexible Element 30 in der körperteilangepassten Form wird es versteift. Dazu wird Luft aus dem flexiblen Element 30, das hier eine luftdichte Hülle 32 aufweist, über den Anschlussschlauch 36 abgesaugt und die Füllung der luftdichten Hülle 32 wird in sich zusammengedrückt. Dabei wird der Verbund von Styroporkugeln und vernetzten Fasern so ineinander verkeilt, dass er sich nicht mehr in der Form verändern lässt. Damit ist die Form des flexiblen Elements nicht mehr veränderbar. Dieser Effekt wird rückgängig gemacht, indem wieder Luft in die luftdichte Hülle 32 zugeführt (Deaktivierung der Versteifungseinrichtung) wird.

Die Enden der Raststreifen 51 werden über ein Zugband, das die Öse 52 mit der Lasche 53 verbindet, gegeneinander verschoben, so dass sich das flexible Element 30 enger an das Körperteil 10 legt und auf das Körperteil 10 drückt. Es kann aber auch ohne Zugband gearbeitet werden, indem das eine Ende des Raststreifens (das mit der Öse 53, die dann aber nicht benötigt wird) direkt durch die Lasche 54 geführt wird und dann durch Zug an diesem Ende die Enden des Raststreifens gegeneinander verschoben werden. Der Druck auf das Körperteil 10 wird durch Festziehen, bzw. Lockern der Raststreifen reguliert.

Das Drucksensorelement 40 wird durch das an das Körperteil 10 gedrückte flexible Element 30 auch an das Körperteil 10 gedrückt und es entsteht eine direkte Kopplung des Drucksensorelements 40 an das Körperteil 10. Daher können pulsatile Signale der in dem Körperteil befindlichen Blutgefäße gemessen werden. Das Drucksensorelement 40 wandelt diese Signale in elektrische Signale um. Die elektrischen Signale werden dann über das Sensorkabel 43 weitergeleitet.

Die Steuereinheit 60 kontrolliert die Versteifung des flexiblen Elements 30 und erfasst die Messwerte des Drucksensorelements 40. Durch die Steuereinrichtung 60 werden auf Basis der verfügbaren Sensorwerte Kennzahlen berechnet, wie zum Beispiel HLI-Parameter, insbesondere HZV, SVV, PPV, PEPV. Diese verfügbaren Sensorwerte und/ oder berechnete Kennzahlen werden durch die Steuereinheit angezeigt - beispielsweise auf einem LCD (nicht dargestellt) - und/ oder gespeichert. Die Steuereinheit stellt auch alle Daten zum Auslesen durch einen durch Kabel oder Funk verbindbaren Rechner (nicht dargestellt) bereit.

Die dargestellte Vorrichtung erlaubt es, pulsatile Signale mit einem dem Stand der Technik gegenüber erhöhten Signal-Rausch-Verhältnis zu messen. Auf der Basis dieser qualitativ hochwertigeren Signale, können aussagekräftige Kennzahlen berechnet werden. Das höhere Signal-Rausch-Verhältnis wird durch die optimierte hydraulische Ankopplung eines oder mehrerer Drucksensorelemente 40 an ein Körperteil 10 möglich. Die hydraulische Ankopplung beruht auf der Möglichkeit, ein flexibles Element 30 durch eine Versteifungseinrichtung 31 versteifen zu können. Dieses flexible Element wird zunächst dem Körperteil 10 angepasst und dann versteift. Die Anpassung an das Körperteil 10 wird durch den Druck des Raststreifens unterstützt 51, so dass das Drucksensorelement 40 an das Körperteil gedrückt wird. Nachdem die Luft aus der luftdichten Hülle 32 evakuiert wurde und somit das flexible Element 30 versteift ist, werden pulsatile Signale nicht mehr durch Absorption in flexiblen Elementen gedämpft und stehen so der Druckmessung im Wesentlichen ungedämpft zur Verfügung.

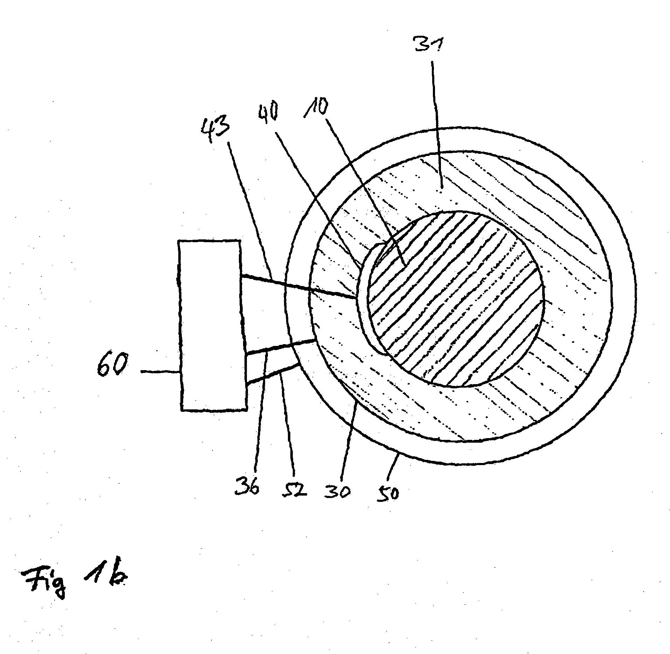

**Figur 1b** zeigt einen schematischen Querschnitt einer erfindungsgemäßen Blutdruckmessvorrichtung in ringförmiger Ausführung und mit einer Blutdruckmanschette als äußere Fixierung.

Das Körperteil 10 ist schraffiert (von links unten nach rechts oben) dargestellt. Dies ist beispielsweise der menschlichen Oberarm. Das flexible Element 30 hat eine ringförmige Gestalt und weißt eine luftdichte Hülle auf. Diese ist mit Styroporkugeln und vernetzten Fasern gleichmäßig gefüllt. Diese versteifbare Füllung ist als Schraffur (von links oben nach rechts unten) abstrahiert und stellt die Versteifungseinrichtung 31 des flexiblen Elements 30 dar. Ein Drucksensorelement 40 ist an der dem Körperteil 10 zugewandten Seite des flexiblen Elements angebracht. In diesem schematischen Querschnitt ist die Anbringung des Drucksensorelements 40 an das flexible Element gezeigt, durch welche das Drucksensorelement mit einer der Oberflächen des flexiblen Elements bündig abschließt. Eine Blutdruckmanschette als äußere Fixierung 50 ist hier in ringförmiger Ausführung dargestellt. Der Durchmesser ist größer als der des flexiblen Elements 30, denn sie ist außen angebracht. Sie verfügt über einen Anschlussschlauch 52 als Steuerleitung, über welchen Luft oder ein Fluid in die Manschette gefüllt und aus der Manschette abgelassen wird. Die restlichen gezeichneten Komponenten entsprechen denen aus Fig. 1a.

Die Anbringung des flexiblen Elements 31 und der Blutdruckmanschette erfolgt durch Überstülpen über das Körperteil 10. Über die Steuerleitung 52 kann der Grad der äußeren Fixierung, also der Druck, den die Blutdruckmanschette 50 auf das Körperteil 10 ausübt, eingestellt werden. Gas oder Flüssigkeit wird in das elastische Element eingefüllt (Druck auf das Körperteil wird erhöht) oder aus dem elastischen Element abgelassen (Druck auf das Körperteil wird vermindert).

Der Vorteil der ringförmigen Ausführungsform ist ein schnelleres Anlegen der Blutdruckmessvorrichtung and das Körperteil. Allerdings ist man hier auf Körperteile beschränkt, die ein Überstülpen des flexiblen Elements 30 erlauben. Zudem muss der Durchmesser des vom flexiblen Element gebildeten Hohlzylinders an das Körperteil grob angepasst sein. Die Verwendung der Blutdruckmanschette ermöglicht eine Variation des Drucks auf das Körperteil, so dass Blutdruck einfach gemessen werden kann.

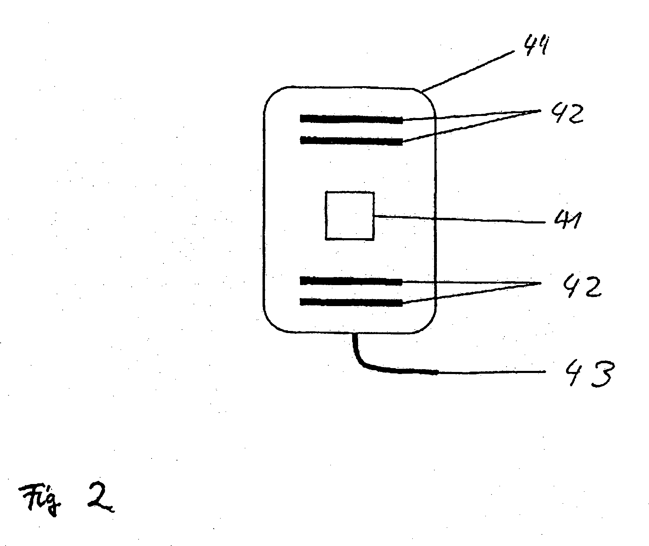

**Figur 2** ist eine Aufsicht auf ein Drucksensorelement, das als Gelkissen ausgeführt ist.

Das Gelkissen 44 ist als abgerundetes Viereck dargestellt. Das Gelkissen ist mit einem Fluid gefüllt. Die Aufsicht geht von dem Spezialfall eines transparenten Gelkissens aus, das mit transparentem Fluid gefüllt ist. So sind Elemente innerhalb des Gelkissens in der Aufsicht sichtbar.

Der in dem Gelkissen 44 eingebettete Drucksensor 41 ist als Viereck in der Mitte des Gelkissens gezeichnet. Der Drucksensor befindet sich im Mittelpunkt des Gelkissens.

In dieser Aufsicht sind auch weitere Sensoren 42 eingezeichnet. Sie sind als dunkle Balken abstrahiert. Die weiteren Sensoren sind Elektroden zur Impedanzmessung. Dabei ist mindestens eine Elektrode als Anregungselektrode und mindestens eine Elektrode als Detektionselektrode vorgesehen.

Die vom Drucksensor 41 und den weiteren Sensoren 42 erzeugten elektrischen Signale werden über das Sensorkabel 43 weitergeleitet.

Das Gelkissen 44 mit den integrierten Sensoren 41, 42 stellt ein Drucksensorelement dar, das bevorzugt an der Innenseite des flexiblen Elements 30 angebracht wird. Daher wird es beim Anlegen des flexiblen Elements 30 auf das Körperteil 10 gedrückt. Der Kontakt, der dabei entsteht, koppelt den in dem Gelkissen 44 befindlichen Drucksensor hydraulisch an das Körpergewebe. Druckoszillationen in einem in dem Körperteil 10 vorhandenen Blutgefäß werden somit über das in dem Gelkissen 44 befindliche Fluid auf den Drucksensor 41 übertragen. Durch die im Vergleich zur Oberfläche des Drucksensors 41 größere Kontaktfläche des Gelkissens 44 zum Körperteil 10 sind die Amplituden der am Drucksensor 41 gemessenen Oszillationen höher als bei direktem Aufliegen des Drucksensors 41 auf dem Körperteil.

Die weiteren am Gelkissen 44 angebrachten Sensoren 42 sind für eine Impedanzmessung ausgelegt. So können über den Hautwiderstand auch pulsatile Signale gemessen werden, da sich der Hautwiderstand mit dem sich durch den Puls ändernden Aderquerschnitt der Blutgefäße ändert. Auch diese Messung findet bevorzugt am Gelkissen 44 statt, da einerseits die direkte Druckmessung mit Hilfe des Drucksensors 41 und die Impedanzmessung aufgrund der örtlichen Übereinstimmung besser miteinander korreliert werden können und andererseits das Gelkissen 44 einen guten Kontakt zum Körperteil gewährleistet.

Die Ausgestaltung eines Drucksensorelements als sensorbestücktes Gelkissen bietet daher vielfältige Messvarianten mit gleichzeitig gutem Kontakt zum Körperteil.

**Figur 3a** zeigt ein Schema einer bevorzugten Ausführungsform der Versteifungseinrichtung des flexiblen Elements in Form einer mit Reiskörnern und Stoffgewebe und Wollfasern gefüllten luftdichten Hülle.

Ein Ausschnitt der luftdichten Hülle 32 ist durch eine dicke schwarze Begrenzungslinie jeweils nach oben und unten angedeutet. In dieser Ausführungsform sind in der Füllung der luftdichten Hülle 32 Elemente aus nicht komprimierbarem Material, also im Wesentlichen inkompressible Elemente 331 enthalten. Diese sind als Kreise bzw. Ellipsen gekennzeichnet. Es handelt sich hier um Reiskörner. Eine dünne gepunktete Linie stellt ein Stoffgewebe 342 als weitere Komponente des Füllmaterials dar. Wollfasern 341 sind als schwarze, gebogene, ineinander verhakte kurze Linien dargestellt. Auch sie sind Teil der Füllung.

In dieser Ausführung liegt ein Schichtaufbau der Füllung vor. Die Reiskörner 331 sind in Lagen von Wollfasern 341 und Stoffgewebe 342 eingebettet. Durch Evakuieren der in der luftdichten Hülle 32 befindlichen Luft werden die komprimierbaren Teile der Füllung komprimiert. Die Reiskörner werden nicht komprimiert sondern pressen sich aneinander und verkeilen sich in den anliegenden Schichten. Damit wird die luftdichte Hülle 32 nicht mehr eindrückbar.

Durch Evakuierung der luftdichten Hülle kann daher eine hohe Endfestigkeit erreicht werden. Je höher die Endfestigkeit umso günstiger wird das Signal-Rausch Verhältnis.

**Figur 3b** zeigt ein Schema einer bevorzugten Ausführungsform der Versteifungseinrichtung des flexiblen Elements in Form einer mit Plastikgranulat, Kunststoffnetzen und Baumwollfasern gefüllten luftdichten Hülle.

Der Unterschied zur vorigen Figur ist, dass das Füllmaterial nun aus im Wesentlichen inkompressiblem Plastikgranulat 331, Kunststoffnetzen 343 sowie Baumwollfasern 341 besteht. Zudem ist die Schichtung der Füllstoffe in einer anderen Reihenfolge erfolgt.

Die Verwendung von Plastikgranulat ist eine kostengünstige Möglichkeit der Füllung. Zudem kann man bei der Herstellung die Größe des Plastikgranulats auf die Dimensionen des anzupassenden Körperteils abstimmen.

**Figur 3c** zeigt ein Schema einer bevorzugten Ausführungsform der Versteifungseinrichtung des flexiblen Elements in Form eines Papierstapels.

Die Figur zeigt einen Stapel Papier aus mehreren übereinander gelegten Blättern 311.

Im nicht gebogenen Zustand ist dieser Stapel flexibel. Wird er jedoch innen in eine konventionelle Blutdruckmanschette eingelegt und um beispielsweise den Oberarm mit der Blutdruckmanschette gewickelt, so wirkt der Stapel Papier versteifend. Er lässt sich durch die Pulsschwankungen des Arms dann nicht mehr eindrükken. Praktisch handelt es sich um eine auf der Innenseite mit mehreren Lagen Papier versehenen Blutdruckmanschette. Ein zwischen dem Arm und dem Stapel Papier befindliches Druckelement kann so Signale von hervorragender Signalqualität messen. Der Papierstapel kann auch in einer Hülle geschützt liegen.

In einem weiteren Ausführungsbeispiel kann auch die luftdichte Hülle mit mehreren Lagen aus Papier oder aus einem ähnlichen Material gefüllt sein. Dadurch dass die Lagen Papier nahezu ohne Zwischenraum aufeinanderliegen, ist die Hülle bereits ohne eine Evakuierung bereits sehr Druckfest und bietet gute Eigenschaften für eine Blutdruckmessung. Wird nun die luftdichte Hülle zusätzlich noch evakuiert, bilden die Lagen Papier einen festen Verbund dessen Steifigkeit weiter zunimmt.

**Figur 3d** zeigt ein Schema einer bevorzugten Ausführungsform der Versteifungseinrichtung des flexiblen Elements in Form einer mit Styroporkugeln und Fasern gefüllten luftdichten Hülle.

Ein Ausschnitt der luftdichten Hülle 32 ist durch eine dicke schwarze Begrenzungslinie jeweils nach oben und unten angedeutet.

Die Styroporkugeln 33 sind als schwarz umrandete Kreise dargestellt. Sie weisen verschiedene Radien auf.

Die Fasern 34 sind als teilweise gekräuselte Linien gezeichnet. Die Anordnung der Fasern ist eine zufällige Vernetzung. Klebstofftröpfchen 35 sind an einigen Vernetzungspunkten angebracht. Diese sind als schwarze Kreise gekennzeichnet.

Das Gemisch aus den Komponenten 33-35 befindet sich in der luftdichten Hülle. Die Styroporkugeln 33 sind gleichmäßig in der fliesartigen Vernetzung der Fasern 34 mit den Klebstofftröpfchen 35 verteilt. Luft befindet sich zwischen den einzelnen Elementen des Gemischs. Die luftdichte Hülle 32 ist aus einem Material, das bevorzugt flexibel und luftundurchlässig ist. Befindet sich Luft in der luftdichten Hülle 32 und zwischen den Styropörkugeln 33, lässt sich die luftdichte Hülle 32 mitsamt des Gemisches verformen.

Wird die Luft aus der luftdichten Hülle 32 evakuiert, so zieht sich die luftdichte Hülle 32 zusammen und drückt das in ihr befindliche Gemisch zusammen. Die Styroporkugeln 33 und die Fasern 34 legen sich eng aneinander und verkeilen sich. Aufgrund der hohen Haftreibung, die zwischen den einzelnen Elementen durch den gegenseitigen Druck entsteht, lässt sich das Gemisch nicht mehr verformen und wird steif. Daher wird auch die luftdichte Hülle 32 steif. Das flexible Element 30 wird auch versteift, da die luftdichte Hülle 32 die in das flexible Element 30 integrierte Versteifungseinrichtung darstellt.

Sobald wieder Luft in die luftdichte Hülle 32 eingelassen wird, kann die luftdichte Hülle 32 - und damit das flexible Element 30 - wieder verformt werden.

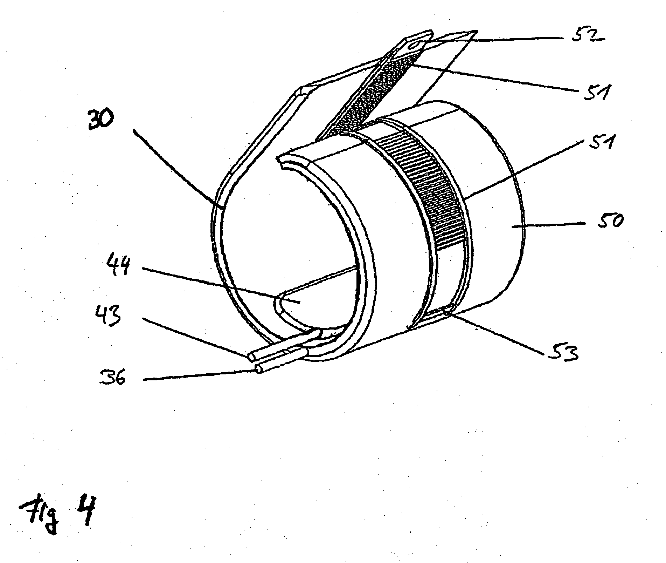

Die hier gezeigte, mit Styroporkugeln 33 und mit bevorzugt durch Klebstofftröpfchen 35 vernetzten Fasern 34 gefüllte luftdichte Hülle 32 stellt eine Versteifungseinrichtung dar. Sie lässt sich beispielsweise über einen Anschlussschlauch aktivieren (evakuieren der Luft in der luftdichten Hülle) und auch wieder deaktivieren (einlassen von Luft in die luftdichte Hülle). Sie ist geeignet, das flexible Element 30 zu versteifen und wieder in einen flexiblen Zustand zu überführen. **Figur 4** zeigt eine dreidimensionale Zeichnung einer bevorzugten Ausführungsform der erfindungsgemäßen Blutdruckmessvorrichtung mit Blutdruckmanschette, Gelkissen und Raststreifen.

Ein Gelkissen 44 ist als Drucksensorelement an dem flexiblen Element 30 angebracht. Ein Sensorkabel 43 und eine Steuerleitung für das flexible Element 36 werden aus der Vorrichtung herausgeführt.

Eine Blutdruckmanschette ist als äußere Fixierung 50 um das flexible Element 30 angebracht, welche eine Fixierung des flexiblen Elements 30 an ein Körperteil ermöglicht.

Ein innerer bzw. äußerer Raststreifen 51, mit einer Öse 52 bzw. einer Lasche 53 sind zudem an dem flexiblen Element 30 bzw. an der äußeren Fixierung angebracht.

Beim Anlegen wird das Körperteil 10 mit dem Verbund aus flexiblem Element 30, Gelkissen 44 Blutdruckmanschette 50 und Raststreifen 51 umwickelt, so dass sich das flexible Element 30 dem Körperteil 10 anpasst, und es wird durch die Manschette 50 und/ oder die Raststreifen 51 fixiert. Bevorzugt wird der Druck, den die äußere Fixierung 50 und/ oder die Raststreifen 51 auf das Körperteil ausüben, gezielt variiert, so dass verschiedenste Messungen pulsatiler Signale durchgeführt werden können. Durch das Versteifen des flexiblen Elements 30 erhält das Körperteil eine starre Umschalung und das Drucksensorelement 40 ist rigide an das Körperteil gekoppelt. Diese Kopplung ist hydraulisch sehr vorteilhaft, da Energie der pulsatilen Signale nicht an das im versteiften Zustand befindliche flexible Element 30 weitergegeben wird und somit nicht für die Messung verloren geht.

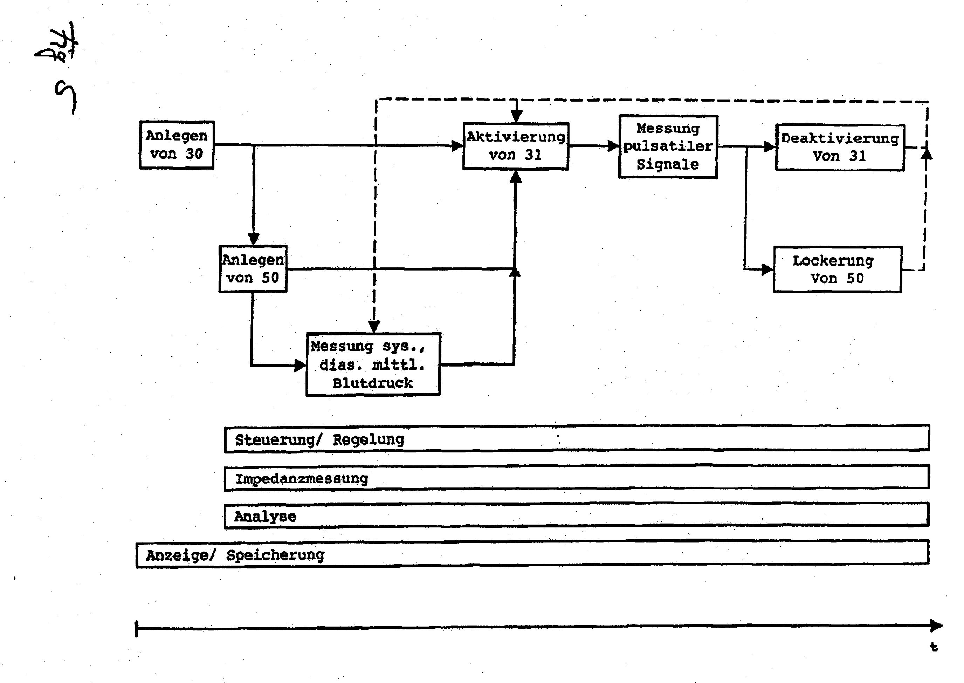

**Figur 5** zeigt ein schematisches Ablaufdiagramm erfindungsgemäßer Verfahren.

Das Diagramm besteht aus beschrifteten Blöcken und Pfeilen. Die Blöcke bezeichnen Verfahrensschritte. Die zeitliche Abhängigkeit von Verfahrenschritten relativ zueinander ist, wenn maßgeblich, durch Pfeile angegeben. Dabei ist ein Verfahrensschritt, der nach einem anderen Verfahrensschritt ausgeführt wird, durch einen Pfeil verbunden, der zu dem späteren Verfahrensschritt zeigt. Verfahrenschritte ohne Pfeilverbindungen haben keine zwingende zeitliche Abhängigkeit zu anderen Verfahrensschritte. Die eingezeichnete Abszisse kann dementsprechend als Zeitachse angesehen werden. Blöcke, die mindestens eine gemeinsame, vertikale Schnittachse aufweisen, sind in dem Überschneidungsbereich parallel ausführbar. Sind Blöcke genau untereinander angeordnet, so können die entsprechenden Verfahrensschritte gleichzeitig oder in beliebiger Reihenfolge zueinander ausgeführt werden. Die Blöcke sind so angeordnet, dass ein Verfahrensablauf von links beginnend nach rechts durchlaufen wird. Pfeile, die entgegen der normalen Zeitrichtung eingezeichnet sind, bedeuten keineswegs eine Umkehr der Zeit oder ein Zurückgehen in die Vergangenheit. Diese Pfeile deuten an, dass in bevorzugten Verfahren einzelne Schritte mehrfach ausgeführt werden können oder dass ein ganzer Abschnitt des Verfahrens wiederholbar ist. Diese Pfeile sind daher gestrichelt dargestellt. Erstreckt sich ein Block über einen längeren Bereich der Zeitachse, so bedeutet dies, dass der entsprechende Verfahrensschritt bevorzugt in dem abgedeckten Zeitraum ausgeführt wird, beginnend von dem Zeitpunkt, der durch die linke Begrenzung des Blocks angedeutet ist. Die durch die einzelnen Verfahrensschritte abgedeckten Zeiträume sind nicht maßstäblich gezeichnet. Das Diagramm gibt keine Information über die wirklichen Ausführungszeiten, oder die relative Länge der Ausführungszeiten einzelner Verfahrensschritte zueinander, sondern visualisiert die zeitliche Reihenfolge bzw. Abfolge und die Parallelität der erfindungsgemäßen Verfahrensschritte.

Das erfindungsgemäße Verfahren beginnt mit dem Anlegen des flexiblem Elements 30 an das für die Messung bestimmte Körperteil 10. Dabei wird das flexible Element 30 so angelegt, dass es sich an das Körperteil 10 anpasst. Dies ist durch die Verformbarkeit des flexiblen Elements 30 möglich, die es aufweist, solange die Versteifungseinrichtung 31 des flexiblen Elements 30 nicht aktiviert ist. Durch das körperteilangepasste Anlegen des flexiblen Elements 30 werden auch die an der dem Körperteil 10 zugewandten Seite des flexiblen Elements 30 angebrachten Drucksensorelemente 40 an das Körperteil 10 angepasst. Dieser Verfahrensschritt ist durch den Block "Anlegen von 30" gekennzeichnet.

Der zweite Schritt des erfindungsgemäßen Verfahrens ist dann das Aktivieren der Versteifungseinrichtung 31 bzw. das Versteifen des flexiblen Elements 30. Das flexible Element 30 ist dann nicht mehr verformbar. Es kann so keine Druckschwankungen in sich aufnehmen oder weitergeben. Dieser Verfahrensschritt ist durch den Block "Aktivierung von 31" gekennzeichnet.

Der dritte Schritt des erfindungsgemäßen Verfahrens besteht dann im Messen der pulsatilen Signale über einen bestimmten Zeitraum. Die am flexiblen Element 30 angebrachten Drucksensorelemente 40 sind hydraulisch optimiert mit dem Körperteil 10 verbunden, denn das flexible Element 30 bildet eine starre äußere Hülle um die angebrachten Drucksensorelement 40, so dass sich vom Körperteil 10 ausgehende Druckschwankungen nicht in weitere Komponenten der Blutdruckmessvorrichtung fortpflanzen und somit keine Energie verlieren, die dann nicht mehr in das durch das jeweilige Drucksensorelement 40 gemessene Signal mit einfließen könnte. Der Messzeitraum umfasst dabei z.B. drei respiratorische Zyklen. Die Druckschwankungen werden durch die an dem flexiblen Element 30 angebrachten Drucksensorelemente 40 in elektrische Signale umgewandelt. Dieser Verfahrensschritt ist durch den Block "Messung pulsatiler Signale" gekennzeichnet.

In einem dritten Schritt wird die Versteifungseinrichtung 31 des flexiblen Elements 30 wieder deaktiviert. Bei einer mit Styroporkugeln 33 und vernetzten Fasern 34 gefüllten luftdichten Hülle 32 erfolgt die Deaktivierung durch Belüften der luftdichten Hülle 32. Dieser Verfahrensschritt ist durch den Block "Deaktivierung von 31" gekennzeichnet.

Der Verfahrensblock "Anlegen von 50" deutet an, dass ein bevorzugtes weiteres Verfahren zusätzlich den Verfahrenschritt des Anlegens einer äußeren Fixierung 50 um das flexible Element 30 enthält. Dies erfolgt bevorzugt nach dem Anlegen des flexiblen Elements 30 an das Körperteil 10, wie im Diagramm dargestellt. Mit dem Anlegen der äußeren Fixierung 50 wird auch der Fixierungsgrad der äußeren Fixierung gezielt eingestellt. Bevorzugt wird der Fixierungsgrad so eingestellt, dass die äußere Fixierung 50 über das flexible Element 30 einen Druck auf das Körperteil 10 ausübt, der im pulsatilen Bereich liegt, also zwischen dem systolischen und dem diastolischen Blutdruck. Damit wird bevorzugt durch den darauf folgenden Verfahrensschritt das flexible Element im pulsatilen Bereich versteift.

Der Verfahrensblock "Lockerung von 50" deutet an, dass bei Verwendung einer äußeren Fixierung 50 die äußere Fixierung 50 nach der Messung wieder gelockert wird. Dies verhindert eine Schädigung des Körperteils 10.

Der Verfahrensblock "Messung sys., dias., mittl. Blutdruck" deutet an, dass bei einem weiteren bevorzugten Verfahren systolischer und/ oder diastolischer und/ oder mittlerer Blutdruck gemessen wird. Diese Messung findet dann statt, wenn sich das flexible Element 30 noch nicht in dem versteiften Zustand befindet, so wie im Diagramm angegeben. Durch die äußere Fixierung 50 kann der auf das Körperteil 10 wirkende Druck dabei gezielt variiert werden, so dass eine oszillatorische Messung der genannten Drücke erfolgen kann.

Der breite Verfahrensblock "Steuerung/ Regelung" deutet an, dass bei weiteren bevorzugten Verfahren ab dem Zeitpunkt, an dem das flexible Element 30 angelegt ist, eine Steuerung und/oder Regelung der Versteifungseinrichtung 31 und/ oder des Fixierungsgrads der äußeren Fixierung 51 zu jedem nachfolgendem Zeitpunkt oder nur zeitweise stattfindet.

Der breite Verfahrensblock "Impedanzmessung" deutet an, dass bei einem weiteren bevorzugten Verfahren ab dem Zeitpunkt, an dem das flexible Element 30 angelegt ist, die Messung des Gewebewiderstands vorgesehen ist. Die Messung der Impedanz erfolgt bevorzugt zu einem oder mehreren Zeitpunkten, besonders bevorzugt alternierend zur Messung der pulsatilen Signale durch ein Drucksensorelement. In einem bevorzugten Verfahren wird beispielsweise der Blutfluss über eine Impedanzmessung über drei Minuten durchgeführt. Hierbei wird durch die Blutdruckmessvorrichtung im Wesentlichen kein Druck auf das Köperteil ausgeübt. Das Blut kann im Wesentlichen ungehindert fließen. Dann werden über eine Minute pulsatile Signale über den Drucksensor gemessen. Dabei wird durch die äußere Fixierung ein für die Messung interessanter Druckbereich eingestellt. Der Blutfluss wird interpoliert. Anschließend erfolgt wiederum eine Messung des Blutflusses über die Impedanzmessung, wobei die Blutdruckmessvorrichtung wiederum im Wesentlichen keinen Druck auf das Körperteil ausübt.

Der breite Verfahrensblock "Analyse" deutet an, dass bei einem weiteren bevorzugten Verfahren ab dem Zeitpunkt, an dem das flexible Element 30 angelegt ist, eine Analyse der gemessen Daten vorgesehen ist. Die Analyse erfolgt besonders bevorzugt zu einem oder mehreren Zeitpunkten. Es kann beispielsweise die Schlagvolumenvariation (SVV) kontinuierlich aus der pulsatilen Blutflussmessung (per Impedanz) berechnet werden. Die PPV, das Schlagvolumen (SV), Herzrate (HR), Herzzeitvolumen (HZV = HR x SV), dP/dtmax sowie weitere Parameter können aus der Druckmessung und Pulskontur berechnet werden. Der breite Verfahrensblock "Anzeige/ Speicherung" deutet an, dass bei einem weiteren bevorzugten Verfahren verfügbare Signale und/ oder Parameter angezeigt und/ oder gespeichert werden. Dies ist kontinuierlich ab dem Zeitpunkt ihrer Verfügbarkeit möglich, erfolgt bevorzugt aber erst zu einem bestimmten Zeitpunkt, besonders bevorzugt zu mehreren Zeitpunkten.

Die Verfahren können in Teilen auch wiederholt ausgeführt werden. So ist es möglich, nach der Deaktivierung der Versteifungseinrichtung 31 und - wenn eine äußere Fixierung 50 verwendet wird - bevorzugt nach der Lockerung der äußeren Fixierung 50 mit dem Versteifen des flexiblen Elements 30 oder mit der oszillatorischen Blutdruckmessung fortzufahren. Die gestrichelten Rückwärtspfeile geben hierbei die bevorzugten Ablaufpfade an.

Durch das Diagramm in Fig. 5 wird anschaulich dargestellt, in welcher Weise die verschiedenen Verfahrensschritte im Ablauf des erfindungsgemäßen Verfahrens und im Ablauf weiterer erfindungsgemäßer Verfahren zusammenhängen.

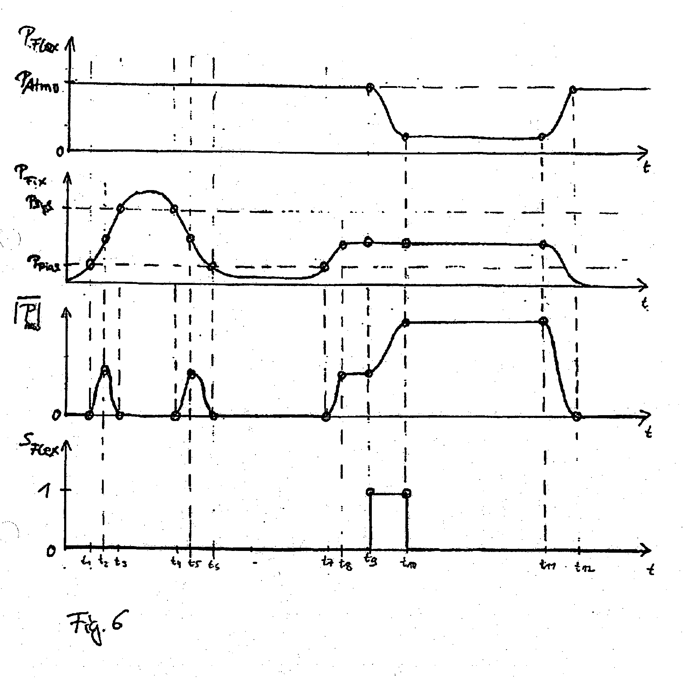

Figur 6 zeigt den zeitlichen Verlauf ausgewählter Zustandsgrößen während einer beispielhaften Anwendung der Blutdruckmessvorrichtung.

Es sind vier Koordinatensysteme eingezeichnet. Die x-Achse ist jeweils die Zeit t. Das oberste zeigt den Verlauf des Luftdrucks $P_{Flex}$ in dem flexiblen Element 30 relativ zum atmosphärischen Umgebungsdruck $P_{Atmo}$. Das zweite Diagramm von oben zeigt den Druck $P_{Fix}$ der äußeren Fixierung 50 auf das flexible Element 30 und damit das Körperteil 10 relativ zu den Werten des systolischen bzw. diastolischen Blutdrucks $P_{Dias}$ bzw. $P_{Sys}$. Das dritte Achsensystem von oben zeigt den Verlauf von $|P_{Mess}|$. Dies ist der über mindestens einen respiratorischen Atemzyklus gemittelte Betrag der Amplitude des durch das Drucksensorelement 40 gemessenen Drucks. Das unterste Diagramm zeigt den Verlauf eines Steuersignals $S_{Flex}$ zur Aktivierung der Versteifungseinrichtung 31. Über dieses Signal wird beispielsweise eine Pumpe gesteuert. Der Wert 0 bedeutet "aus" der Wert 1 bedeutet "an".

Zu Beginn wird der systolische, diastolische und der mittlere Blutdruck ermittelt. Hierzu wird $P_{Fix}$ erhöht. Ab dem Zeitpunkt $t_1$ werden pulsatile Signale durch das Drucksensorelement gemessen, so dass auch $|P_{Mess}|$ steigt. Bei einem Zeitpunkt $t_2$, an welchem $P_{Fix}$ den Wert des Blutmitteldrucks durchwandert, weist $|P_{Mess}|$ ein lokales Maximum auf. Zum Zeitpunkt $t_3$ überschreitet $P_{Fix}$ den Wert des systolischen Blutdrucks und $|P_{Mess}|$ ist wiederum 0. Wird $P_{Fix}$ wieder verringert, treten pulsatile Signale und damit $|P_{Mess}|>0$ zwischen den Zeitpunkten $t_4$ und $t_6$ auf, mit einem lokalen Maximum von $|P_{Mess}|$ bei $t_5$. Aus diesem Messverlauf kann der systolische, diastolische und mittlere Blutdruck ermittelt werden. Danach wird $P_{Fix}$ vom Zeitpunkt $t_7$ zum Zeitpunkt $t_8$ erhöht, so dass ab $t_8$ der Druck $P_{Fix}$ auf einen Wert nahe dem mittleren Blutdruck eingestellt ist. $|P_{Mess}|$ stellt sich auf einen Wert ein, der gleich oder kleiner des Wertes der bereits erreichten lokalen Maxima zur Zeit $t_2$ und $t_5$ ist. Zum Zeitpunkt $t_9$ wird das Steuersignal $S_{Flex}$ von 0 auf 1 erhöht, d.h. das flexible Element 30 wird versteift. Der Übergang in den versteiften Zustand dauert von $t_9$ bis $t_{10}$ an. In dieser Zeit wird Luft aus dem flexiblen Element evakuiert. Der Druck in dem flexiblen Element $P_{Flex}$ fällt unter den Umgebungsdruck $P_{Atmo}$. Mit dem Fortschreiten des Evakuierens wird die hydraulische Kopplung des Drucksensorelements 40 an das Körperteil 10 verbessert, so dass die Signalqualität steigt - und damit auch $|P_{Mess}|$ über einen Wert der zuvor erreichten lokalen Maxima, d.h. dass das Signal-Rausch-Verhältnis verbessert wird. Zum Zeitpunkt $t_{10}$ hat $P_{Flex}$ einen vorbestimmten niedrigen Wert erreicht und die Pumpe wird ausgeschaltet, d.h. $S_{Flex}$ wird von 1 auf 0 gesetzt, und das flexible Element wird durch ein Ventil abgedichtet. Zwischen $t_{10}$ und $t_{11}$ werden die pulsatilen Signale dann mit sehr guter Signalqualität gemessen. Zum Zeitpunkt $t_{11}$ wird die äußere Fixierung 50 wieder gelockert und das flexible Element 30 durch Öffnen des entsprechenden Ventils wieder belüftet, so dass die Werte von $P_{Flex}$, $P_{Fix}$ und $|P_{Mess}|$ bis zum Zeitpunkt $t_{12}$ wieder in ihren Anfangszustand zurückwandern.

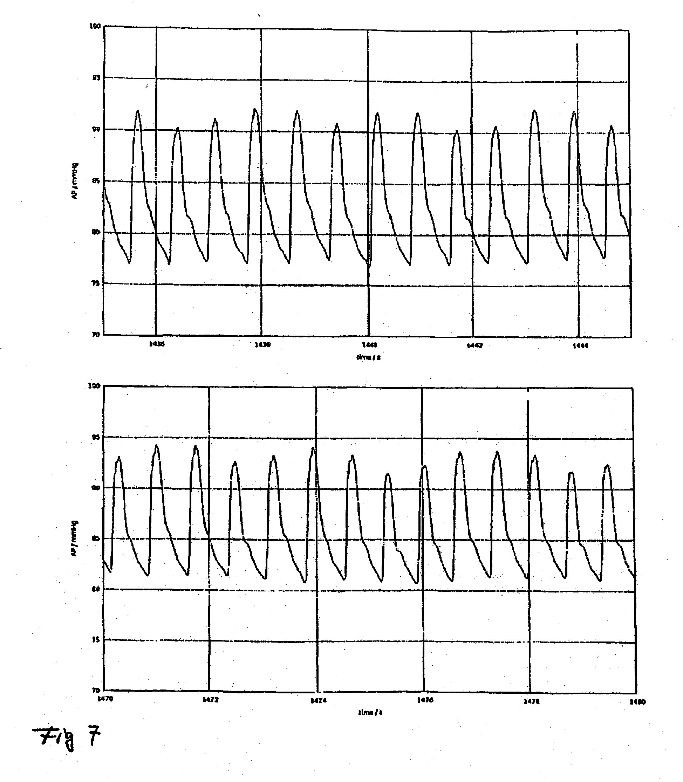

Figur 7 zeigt den zeitlichen Verlauf des durch einen Drucksensor gemessenen Druckverlaufs bei Ausführung der Blutdruckmessvorrichtung mit einer äußeren Fixierung, die das flexible Element nicht vollständig an allen Enden überlappt.

Das flexible Element besteht in dieser Ausführung aus einer mit einem Papierstapel von 40 Blatt Papier mit 80 g/m$^2$ gefüllten, evakuierbaren, luftdichten Hülle. Dieses flexible Element liegt in einer konventionellen Blutdruckmanschette und ist mit dieser um den Oberarm gewickelt. Die Blutdruckmanschette überlappt jedoch nicht das flexible Element in Längsrichtung des Arms, sondern das flexible Element steht aus der Blutdruckmanschette hervor. Ein YoYo-Drucksensor (TM der Firma UP-Med) liegt als Drucksensorelement zwischen dem flexiblen Element und der Haut.

In dieser Figur sind nun zwei Graphen gezeigt. Der untere Graph beschreibt den mit einem elektronischen High-Fidelity-Drucksensor (in diesem Fall ein YoYo-Drucksensor, TM der Firma UP-Med) gemessenen Druckverlauf bei belüfteter luftdichter Hülle und der obere Graph den Druckverlauf bei evakuierter luftdichter Hülle. Man sieht deutlich den Verlauf des Pulsschlags. Die untere Kurve (belüftete Hülle) zeigt abgerissene Peaks und Störungen im Signal. Der obere Signalverlauf (evakuierte Hülle) weist diese Störungen nicht mehr so deutlich auf. Generell ist das Signal beider Graphen aber ein wenig verwaschen, denn die Nebenpeaks sind nicht sehr deutlich zu erkennen.

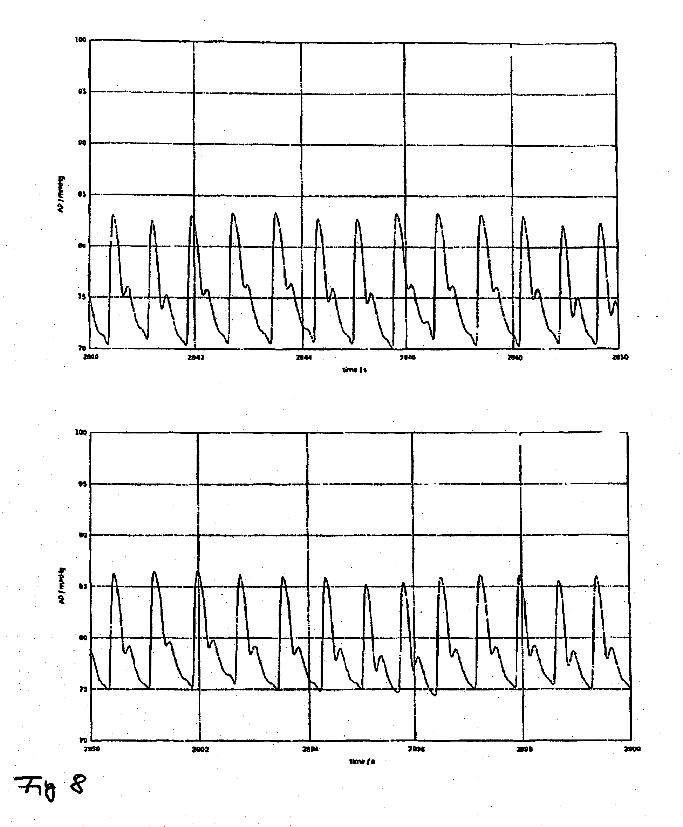

Figur 8 zeigt den zeitlichen Verlauf des durch einen Drucksensor gemessenen Druckverlaufs bei Ausführung der Blutdruckmessvorrichtung mit einer äußeren Fixierung, die das flexible Element vollständig an allen Enden überlappt.

Der Unterschied in der Ausführung der Blutdruckmessvorrichtung zu der in der vorigen Figur genannten Blutdruckmessvorrichtung ist, dass nun die konventionelle Manschette, den in der luftdichten Hülle befindlichen Papierstapel komplett überdeckt.

Auch hier sieht man in zwei Graphen die gemessenen Drucksignale für den Fall einer evakuierten Hülle (oben) und einer belüfteten Hülle (unten). Es fällt auf, dass mit dieser Ausführungsform qualitativ sehr hochwertige Signale gemessen werden. Der Unterschied zwischen der evakuierten luftdichten Hülle und der belüfteten ist nicht mehr sehr groß. Jedoch sind die Nebenpeaks im oberen Graphen (evakuierte Hülle) deutlicher zu erkennen. Die bereits sehr hohe Signalqualität bei nicht evakuierter Hülle steht mit dem schon im nicht evakuierten Zustand sehr druckfesten Stapel Papier in Verbindung.

Bezugszeichenliste

| | |
|---|---|
| 10 | Körperteil |
| 20 | Blutdruckmessvorrichtung |
| 30 | Flexibles Element |
| 31 | Versteifungseinrichtung |
| 311 | Papierblatt |
| 32 | Luftdichte Hülle |
| 33 | Styroporkugeln |
| 331 | Inkompressible Elemente |
| 34 | Fasern |
| 341 | (Baum-)Wollfasern |
| 342 | Stoffgewebe |
| 343 | Kunststoffnetz |
| 35 | Klebstofftröpfchen |
| 36 | Steuerleitung für das flexible Element |
| 40 | Drucksensorelement |
| 41 | Drucksensor |
| 42 | Elektroden |
| 43 | Sensorkabel |
| 44 | Gelkissen |
| 50 | Äußere Fixierung |
| 51 | Raststreifen |
| 52 | Steuerleitung für die äußere Fixierung |
| 53 | Öse |
| 54 | Lasche |
| 60 | Steuereinrichtung |

(fortgesetzt)

| | |
|---|---|
| | |

Aspekte

1. Blutdruckmessvorrichtung (20) umfassend ein flexibles Element (30), das eingerichtet ist, ein Körperteil (10) zumindest teilweise zu umgeben dadurch gekennzeichnet, dass an dem flexiblen Element (30) mindestens ein Drucksensorelement (40) angebracht ist und das flexible Element (30) eine Versteifungseinrichtung (31) aufweist, durch die das flexible Element (30) versteifbar ist.

2. Blutdruckmessvorrichtung (20) nach Aspekt 1, dadurch gekennzeichnet, dass das Drucksensorelement (40) ein in ein Gelkissen (44) eingebetteter Drucksensor (41) ist.

3. Blutdruckmessvorrichtung (20) nach einem oder mehreren der vorhergehenden Aspekte, dadurch gekennzeichnet, dass zusätzlich weitere Sensoren vorgesehen sind, insbesondere eine Sensoreinrichtung zur Messung der Impedanz und/oder Elektroden (42).

4. Blutdruckmessvorrichtung (20) nach einem oder mehreren der vorhergehenden Aspekte, dadurch gekennzeichnet, dass eine äußere Fixierung (50) vorgesehen ist, die zumindest teilweise um das flexible Element (30) angeordnet ist/anordenbar ist.

5. Blutdruckmessvorrichtung (20) nach einem oder mehreren der vorhergehenden Aspekte, dadurch gekennzeichnet, dass das Drucksensorelement (40) ein Sensor oder eine Kombination von Sensoren aus der Menge folgender Sensoren ist: Elektroden (42) zur Impedanz- und/ oder Potentialmessung, photoelektrische Sensoren, kapazitive Sensoren, Beschleunigungssensoren.

6. Blutdruckmessvorrichtung (20) nach einem oder mehreren der vorhergehenden Aspekte, dadurch gekennzeichnet, dass die Versteifungseinrichtung (31) mindestens eine luftdichte Hülle (32) mit mindestens einem Anschlussschlauch (36) aufweist.

7. Blutdruckmessvorrichtung (20) nach Aspekt 6, dadurch gekennzeichnet, dass die luftdichte Hülle (32) im Wesentlichen inkompressible Elemente (331) aufweist, deren Volumen sich in einem Vakuum um weniger als 50% gegenüber dem Volumen bei Atmosphärendruck ändert.

8. Blutdruckmessvorrichtung (20) nach einem oder mehreren der Aspekte 6 bis 7, dadurch gekennzeichnet, dass die luftdichte Hülle (32) vernetzte Fasern (34) aufweist.

9. Blutdruckmessvorrichtung (20) nach einem oder mehreren der Aspekte 6 bis 8, dadurch gekennzeichnet, dass die luftdichte Hülle (32) Styroporkugeln (33).

10. Blutdruckmessvorrichtung (20) nach einem oder mehreren der vorhergehenden Aspekte, dadurch gekennzeichnet, dass die Versteifungseinrichtung (31) einen Stapel von Blättern aus Papier (311) oder aus einem ähnlichen Material aufweist.

11. Blutdruckmessvorrichtung (20) nach einem oder mehreren der vorhergehenden Aspekte, dadurch gekennzeichnet, dass die Blutdruckmessvorrichtung (20) mindestens einen Raststreifen (51) aufweist.

12. Blutdruckmessvorrichtung (20) nach einem oder mehreren der vorhergehenden Aspekte, dadurch gekennzeichnet, dass eine Steuereinrichtung (60) zur Steuerung der Versteifungseinrichtung (31) vorgesehen ist.

13. Blutdruckmessvorrichtung (20) nach einem oder mehreren der vorhergehenden Aspekte, dadurch gekennzeichnet, dass eine Analyseeinrichtung zur Analyse und/ oder Anzeige und/ oder Speicherung von Messdaten vorgesehen ist.

14. Verfahren zur Blutdruckmessung eines Lebewesens umfassend die Schritte:

Anlegen eines flexiblen Elements (30) mit einem Drucksensorelement (40) an ein für die Messung relevantes Körperteil (10), so dass das flexible Element (30) eine körperteilangepasste Form einnimmt,

Versteifen des flexiblen Elements (30) in der körperteilangepassten Form,

Messung des Drucksignals über einen bestimmten Zeitraum während sich das flexible Element (30) in dem versteiften Zustand befindet,

Überführung des Zustands des flexiblen Elements (30) zurück in den flexiblen Zustand.

15. Verfahren nach einem oder mehreren der vorhergehenden, auf ein Verfahren bezogenen Aspekte,
dadurch gekennzeichnet, dass
eine äußere Fixierung (50) über das flexible Element (30) angebracht wird.

16. Verfahren nach einem oder mehreren der vorhergehenden, auf ein Verfahren bezogenen Aspekte,
dadurch gekennzeichnet, dass
das flexible Element (30) inkompressibel versteift wird.

17. Verfahren nach einem oder mehreren der vorhergehenden, auf ein Verfahren bezogenen Aspekte
dadurch gekennzeichnet, dass
das flexible Element (30) durch Evakuieren der in ihm befindlichen Luft versteift wird.

18. Verfahren nach einem der Aspekte 14 bis 16,
dadurch gekennzeichnet, dass
das flexible Element (30) durch Befüllen mit Druckluft versteift wird.

19. Verfahren nach einem oder mehreren der Aspekte 15 bis 18,
dadurch gekennzeichnet, dass
eine konventionelle Blutdruckmanschette als äußere Fixierung (50) verwendet wird.

20. Verfahren nach Aspekte 17 und 19,
dadurch gekennzeichnet, dass
beim Versteifen des flexiblen Elements (30) die evakuierte Luft in die äußere Blutdruckmanschette gepumpt wird.

21. Verfahren nach Aspekt 19 oder 20,
dadurch gekennzeichnet, dass
der systolische, diastolische und mittlere Blutdruck durch Variation des Drucks in der Blutdruckmanschette gemessen wird.

22. Verfahren nach einem oder mehreren der vorhergehenden, auf ein Verfahren bezogenen Aspekte,
dadurch gekennzeichnet, dass
das flexible Element (30) bei einem arteriellen Druck zwischen dem Mitteldruck und dem diastolischen Druck versteift wird.

23. Verfahren nach einem oder mehreren der vorhergehenden, auf ein Verfahren bezogenen Aspekte,
dadurch gekennzeichnet, dass
eine Steuereinrichtung (60) die Versteifung des flexiblen Elements (30) steuert und/ oder den Grad der Fixierung (50) steuert und/ oder die Sensorwerte erfasst.

24. Verfahren nach einem oder mehreren der vorhergehenden, auf ein Verfahren bezogenen Aspekte,
dadurch gekennzeichnet, dass
zusätzlich die elektrische Impedanz des Körperteils (10) gemessen wird.

25. Verfahren nach einem oder mehreren der vorhergehenden, auf ein Verfahren bezogenen Aspekte,
dadurch gekennzeichnet, dass
während einer Messung und/ oder danach aus einem oder mehreren der gemessenen Druckverläufe die arterielle Kurvenform bestimmt wird.

26. Verfahren nach einem oder mehreren der vorhergehenden, auf ein Verfahren bezogenen Aspekte,
dadurch gekennzeichnet, dass
nach einer bestimmten Dauer die Versteifung des flexiblen Elements (30) rückgängig gemacht wird.

27. Verfahren nach einem oder mehreren der vorhergehenden, auf ein Verfahren bezogenen Aspekte
dadurch gekennzeichnet, dass
nach einer bestimmten Dauer die äußere Fixierung (50) gelockert wird.

**Patentansprüche**

1.  Blutdruckmessvorrichtung (20) umfassend ein flexibles Element (30), das eingerichtet ist, ein Körperteil (10) zumindest teilweise zu umgeben, wobei an dem flexiblen Element (30) mindestens ein Drucksensorelement (40) angebracht ist,
    **dadurch gekennzeichnet, dass** das flexible Element (30) eine Versteifungseinrichtung (31) aufweist, durch die das flexible Element (30) so versteifbar ist, dass das flexible Element (30) nicht mehr verformbar ist.

2.  Blutdruckmessvorrichtung (20) nach Anspruch 1,
    **dadurch gekennzeichnet, dass**
    die Versteifungseinrichtung (31) mindestens eine Hülle (32) aufweist, die vorzugsweise flüssigkeitsdicht, stärker bevorzugt luftdicht ist, und vorzugsweise mindestens einen Anschlussschlauch (36) umfasst.

3.  Blutdruckmessvorrichtung (20) nach Anspruch 2,
    **dadurch gekennzeichnet, dass**
    die luftdichte Hülle (32) im Wesentlichen inkompressible Elemente (331) aufweist, deren Volumen sich in einem Vakuum um weniger als 50% gegenüber dem Volumen bei Atmosphärendruck ändert, wobei die inkompressiblen Elemente (331) vorzugsweise Kunststoffgranulat, Reis, Körper aus Polistyrol oder ähnlichem Kunststoff, Papierfetzen, Papierkügelchen, Papierblätter, Styroporkugeln, Sägemehl, Salz, beliebige Pulver oder ähnliche Elemente, oder aber Mischung hieraus sind.

4. Blutdruckmessvorrichtung (20) nach Anspruch 2 oder 3,
   **dadurch gekennzeichnet, dass**
   die luftdichte Hülle (32) vernetzte Fasern (34) aufweist, wobei die Vernetzung der Fasern vorzugsweise aus einer zufällig überkreuzten Anordnung der Fasern besteht, wobei die Vernetzung vorzugsweise durch Klebstofftröpfchen an den Kreuzungspunkten der Fasern besteht; und/ oder
   die luftdichte Hülle (32) Styroporkugeln (33) und/ oder Papierfetzen aufweist; und/ oder
   die Versteifungseinrichtung (31) einen Stapel von Blättern aus Papier (311) oder aus einem ähnlichen Material aufweist.

5. Blutdruckmessvorrichtung (20) nach einem oder mehreren der vorhergehenden Ansprüche,
   **dadurch gekennzeichnet, dass**
   eine Steuereinrichtung (60) zur Steuerung der Versteifungseinrichtung (31) vorgesehen ist.

6. Blutdruckmessvorrichtung (20) nach einem oder mehreren der vorhergehenden Ansprüche,
   **dadurch gekennzeichnet, dass**
   eine äußere Fixierung (50) vorgesehen ist, die zumindest teilweise um das flexible Element (30) angeordnet ist/ anordenbar ist.

7. Blutdruckmessvorrichtung (20) nach einem oder mehreren der vorhergehenden Ansprüche,
   **dadurch gekennzeichnet, dass**
   eine Analyseeinrichtung zur Analyse und/ oder Anzeige und/ oder Speicherung von Messdaten vorgesehen ist.

8. Verfahren zur Blutdruckmessung eines Lebewesens umfassend die Schritte:

   Anlegen eines flexiblen Elements (30) mit einem Drucksensorelement (40) an ein für die Messung relevantes Körperteil (10), so dass das flexible Element (30) eine körperteilangepasste Form einnimmt,
   Versteifen des flexiblen Elements (30) in der körperteilangepassten Form, so dass das flexible Element (30) nicht mehr verformbar ist,
   Messung des Drucksignals über einen bestimmten Zeitraum während sich das flexible Element (30) in dem versteiften Zustand befindet,

Überführung des Zustands des flexiblen Elements (30) zurück in den flexiblen Zustand.

9. Verfahren nach einem oder mehreren der vorhergehenden, auf ein Verfahren bezogenen Ansprüche,
   **dadurch gekennzeichnet, dass**
   eine äußere Fixierung (50) über das flexible Element (30) angebracht wird, wobei vorzugsweise eine konventionelle Blutdruckmanschette als äußere Fixierung (50) verwendet wird.

10. Verfahren nach einem oder mehreren der vorhergehenden, auf ein Verfahren bezogenen Ansprüche,
    **dadurch gekennzeichnet, dass**
    das flexible Element (30) inkompressibel versteift wird.

11. Verfahren nach einem oder mehreren der vorhergehenden, auf ein Verfahren bezogenen Ansprüche,
    **dadurch gekennzeichnet, dass**
    das flexible Element (30) durch Evakuieren der in ihm befindlichen Luft versteift wird.

12. Verfahren nach Anspruch 11,
    **dadurch gekennzeichnet, dass**
    beim Versteifen des flexiblen Elements (30) die evakuierte Luft in eine Blutdruckmanschette, die als äußere Fixierung verwendet wird, gepumpt wird.

13. Verfahren nach Anspruch 12,
    **dadurch gekennzeichnet, dass**
    der systolische, diastolische und mittlere Blutdruck durch Variation des Drucks in der Blutdruckmanschette gemessen wird.

14. Verfahren nach einem oder mehreren der vorhergehenden, auf ein Verfahren bezogenen Ansprüche,
    **dadurch gekennzeichnet, dass**
    eine Steuereinrichtung (60) die Versteifung des flexiblen Elements (30) steuert und/ oder den Grad der Fixierung (50) steuert und/ oder die Sensorwerte erfasst.

15. Verfahren nach einem oder mehreren der vorhergehenden, auf ein Verfahren bezogenen Ansprüche,
    **dadurch gekennzeichnet, dass**
    zusätzlich die elektrische Impedanz des Körperteils (10) gemessen wird; und/oder
    während einer Messung und/ oder danach aus einem oder mehreren der gemessenen Druckverläufe die arterielle Kurvenform bestimmt wird.

16. Verfahren nach einem oder mehreren der vorhergehenden, auf ein Verfahren bezogenen Ansprüche,
    **dadurch gekennzeichnet, dass**
    nach einer bestimmten Dauer die Versteifung des flexiblen Elements (30) rückgängig gemacht wird; und/ oder

nach einer bestimmten Dauer die äußere Fixierung (50) gelockert wird.

Fig. 1a

Fig 1b

Fig 2

Fig 3a

Fig 3b

Fig 3c

Fig 3d

fig 4

Anlegen von 30

Anlegen von 50

Aktivierung von 31

Messung pulsatiler Signale

Deaktivierung Von 31

Lockerung Von 50

Messung sys., dias. mittl. Blutdruck

Steuerung/ Regelung

Impedanzmessung

Analyse

Anzeige/ Speicherung

t

Fig 5

Fig. 6

Fig 7

33

Fig 8

34

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005187481 A1
- US 5255686 A
- EP 01813187 A